(19) Europäisches Patentamt
European Patent Office
Office européen des brevets

(11) Publication number: **0 255 256 A2**

(12) # EUROPEAN PATENT APPLICATION

(21) Application number: 87306193.1

(22) Date of filing: 14.07.87

(51) Int. Cl.[3]: C 07 K 9/00
A 61 K 37/02, C 12 P 21/04
//(C12P21/00, C12R1:03)

(30) Priority: 30.07.86 US 892027

(43) Date of publication of application:
03.02.88 Bulletin 88/5

(84) Designated Contracting States:
AT BE CH DE ES FR GB GR IT LI LU NL SE

(71) Applicant: SMITHKLINE BECKMAN CORPORATION
One Franklin Plaza P O Box 7929
Philadelphia Pennsylvania 19103(US)

(72) Inventor: Carr, Steven Alfred
395 Margo Lane
Berwyn, PA 19312(US)

(72) Inventor: Mueller, Luciano
1801 Butler Pike, Apt. 157
Conshohocken, PA 19428(US)

(72) Inventor: Shearer, Marcia Cathrine
380 Arden Road
Conshohocken, PA 19428(US)

(72) Inventor: Christensen IV, Siegfried Benjamin
2305 Naudain Street
Philadelphia, PA 19146(US)

(72) Inventor: Nisbet, Louis Joseph
Little Court Cromwell Gdns
Marlow Buckinghamshire(GB)

(72) Inventor: Sitrin, Robert David
237 Emerson Drive
Lafayette Hill, PA 19444(US)

(72) Inventor: Jeffs, Peter Walter
1263 Club House Road
Gladwyn, PA 19035(US)

(72) Inventor: Roberts, Gerald D.
636 Schwenksville Road
Schwenksville, PA 19473(US)

(74) Representative: Waters, David Martin, Dr. et al,
Smith Kline & French Laboratories Ltd. Patent
Department Mundells
Welwyn Garden City Hertfordshire AL7 1EY(GB)

(54) **Antibiotics produced by actinomadura parvosata.**

(57) This invention relates to a new microorganism *Actinomadura parvosata*. This invention also relates to a new glycopeptide antibiotic and its biologically functional components and derivatives produced by this new microorganism, to the processes for the preparation thereof, and to pharmaceutical compositions containing the antibiotic or its bioactive components or derivatives. This invention further relates to animal feed premixes and compositions containing the antibiotic or its bioactive components or derivatives and to a process for enhancing feed utilization efficiency and promoting growth in animals.

EP 0 255 256 A2

# ANTIBIOTICS PRODUCED BY ACTINOMADURA PARVOSATA

## SUMMARY OF THE INVENTION

This invention relates to a new microorganism Actinomadura parvosata. This invention also relates to a new glycopeptide antibiotic and its biologically functional components and derivatives produced by this new microorganism, to the processes for the preparation thereof, and to pharmaceutical compositions containing the antibiotic or its bioactive components or derivatives. This invention further relates to animal feed premixes and compositions containing the antibiotic or its bioactive components or derivatives and to a process for enhancing feed utilization efficiency and promoting growth in animals.

More particularly, this invention relates to a new microorganism denoted as Actinomadura parvosata Shearer sp. nov. ATCC 53463 or an active mutant or derivative thereof. The invention also includes a biologically pure culture of Actinomadura parvosata Shearer sp. nov. ATCC 53463 or an active mutant or derivative thereof. This invention also relates to a new glycopeptide antibiotic AAJ-271 complex prepared by culturing or fermentation of Actinomadura parvosata Shearer sp. nov. ATCC 53463 microorganisms, or an active mutant or derivative thereof.

The antibiotic is designated herein as AAJ-271 complex or SK&F AAJ-271. Also included is an isolated AAJ-271 antibiotic comprising an individual or mixture of the bioactive components of the complex. The bioactive components of the complex can be prepared by chromatographic separation and isolation from the complex and the major components are designated herein as AAJ-271 factors A, $B_1$, $B_2$, $C_1$, $C_2$, $C_3$, $C_4$, and X. The complex can also include hydrolysis products of these components. The compounds of the present invention are useful as antibiotics and are active *in vitro* and *in vivo* against gram positive infections. The compounds can also be used in animal health applications due to their growth promotant activity.

## DETAILED DESCRIPTION OF THE INVENTION

### The Microorganism

The new antibiotic AAJ-271 is a complex of glycopeptides produced by the fermentation of a new microorganism *Actinomadura parvosata* Shearer sp. nov. (SK&F AAJ-271). The above microorganism was isolated from a soil sample collected from along the side of a body of fresh water near Myittanyunt, Burma. *Actinomadura parvosata* Shearer sp. nov. (SK&F AAJ-271) has been deposited in the Budapest Treaty Deposits, American Type Culture Collection, Rockville, Maryland, as the type culture under the accession number ATCC 53463.

Because *A. parvosata* sporulates very poorly, it is best maintained as frozen stock in a freezer at -70°C or in the vapor phase of liquid nitrogen. Of various media tested, the best medium for slant cultures of *A. parvosata* was determined to be thin inorganic salts-starch agar (Difco inorganic salts-starch agar [ISP (International Streptomyces Project) 4], 12 g; Difco Bacto agar, 15 g, available from Difco Laboratories of Detroit,

Michigan, U.S.A.; distilled water, 1L) supplemented with yeast extract at 2 g/L. Morphological observations were made on plates of thin inorganic salts-starch agar and modified thin inorganic salts-starch agar (Difco inorganic salts-starch agar, 12 g; Difco Bacto agar, 15 g; distilled water, 800 ml; soil extract 200 ml; thiamine hydrochloride, 0.5 mg; biotin, 0.25 mg).

Inoculum for biochemical and physiological testing was prepared by adding the contents of a frozen vial (1.0 ml) to a flask of broth (glucose, 10 g; soluble starch, 20 g; yeast extract, 5 g; N-Z amine Type A casein hydrolysate [Sheffield Chem. Co. of Memphis, Tennessee, U.S.A.], 5 g; distilled water 1 L) which was placed on a rotary shaker at 28°C, 250 RPM for three to five days. The culture was harvested by centrifugation and washed three times with sterile distilled water by centrifugation. Because the producing organism tended to grow as large flakes in the shake flasks, the washed culture was macerated in a tissue grinder before being used as inoculum. The biochemical and physiological test samples were incubated at 28°C. Readings of the results were made at various times up to six weeks for the plate media. The tubed media were read at weekly intervals for three weeks.

The modified carbon utilization medium of Pridham and Gottlieb (ISP 9) proved unsuitable for the characterization of A. parvosata because there was too little difference between the positive and negative controls. The medium used to determine carbon source utilization of A. parvosata was the C-2 medium of Nonomura and Ohara (J. Ferment. Technol. 11:887-894, 1971).

Susceptibility of A. parvosata to antibiotics was examined by placing BBL susceptibility disks on nutrient agar plates seeded with A. parvosata. Plates were placed at 4°C for 1 hour to permit diffusion of the antibiotics. Diameters of the zones of inhibition were measured after incubation for 1 week at 28°C.

Morphology. A. parvosata is a filamentous organism that forms a mycelium differentiated into: (1) a substrate mycelium that penetrates the agar and forms a compact layer on top of the agar; and (2) an aerial mycelium that bears chains of conidia. No motile elements were ever observed in the aerial or substrate mycelium.

Substrate mycelium. A. parvosata produces a well developed, non-fragmenting substrate mycelium. The long, moderately to densely branched hyphae are approximately 0.5 µm to 1.0 µm in diameter. No spores were ever observed on the substrate mycelium.

Aerial mycelium. A. parvosata does not produce an aerial mycelium on many media. Following five to seven weeks incubation on modified thin inorganic salts-starch agar, it produces long, branching aerial hyphae which bear chains of sporel. These chains of spores are usually fairly long. Chains of more than 30 spores are common, but short chains of ten spores or less are also usually present. A few of the spore chains appear to be sessile but most are borne on short to relatively long sporophores. Some of the spore chains are hooked, irregularly curved or in tightly closed spirals that resemble sporangia. The most common spore chain morphology, however, is one in which the upper half to two-thirds of the spore chain forms spirals of two to six turns. These spirals may be very regular and open or tightly closed. The spirals may also be somewhat irregular and have a knot-like appearance. The spores may vary greatly in size and shape (round to almost square). This variation is particularly apparent when only a few tufts of spore bearing aerial hyphae are produced following prolonged incubation on suboptimal media. On modified thin inorganic-salts starch agar, a typical spore is ovoid to rectangular and approximately 0.5 to 2.5 µm long and 0.5 to 1.3 µm in diameter. The spores are smooth-walled.

Chemotaxonomy. Purified cell wall preparations of A. parvosata, analyzed by the method of Becker (Becker, B., et al., Appl. Microbiol. 13:236-43, 1965) contained the meso-isomer of 2,6-diaminopimelic acid, alanine, glutamic acid, glucosamine, muramic acid, a major amount of glucose and traces of mannose. Whole-cell hydrolysates analyzed by the method of Lechevalier (Lechevalier, M. P., J. Lab. Clin. Med. 71:934-44, 1968) contained galactose, glucose, mannose, ribose, and madurose. The phospholipids present were phosphatidyl ethanolamine, phosphatidyl methylethanolamine, diphosphatidyl glycerol, phosphatidyl inositol, traces of phosphatidyl inositol mannosides, and an unknown glucosamine-containing phospholipid. Thus A. parvosata has a type III cell wall with a type B sugar pattern (Lechevalier, M.P., et al., Int. J. Syst. Bacteriol. 20:435-43, 1970) and a phospholipid pattern of type PIV (Lechevalier, M.P., et al., Biochem. System. Ecol. 5:249-60, 1977).

Biochemical and Physiological Characteristics.

A. parvosata is a gram positive organism that did not grow under anaerobic conditions. Temperature range for growth was 15°C to 45°C; growth at 15°C and 45°C was usually poor. Tests for production of hydrogen sulfide were weakly positive. Gelatin was hydrolyzed but not liquified. Nitrate was reduced to nitrite. No melanin pigments were produced. Adenine (0.5%), casein, L-tyrosine, elastin and hypoxanthine were hydrolyzed but cellulose (Avicel available from FMC Corporation of Philadelphia, Pennsylvania, U.S.A.), and xanthine were not. Hydrolysis of starch was weak and partial (test was negative with iodine indicator but a partial clearing of the starch was visible when plates were viewed against a light). Catalase, phosphatase, deoxyribonuclease and ribonuclease were produced. Allantoin, arbutin, esculin, and urea were decomposed. Tests for the decomposition of

hippurate were positive following prolonged incubation (8 weeks). No growth occurred in 4% NaCl; growth in 2% NaCl was consistent; growth in 3% NaCl was inconsistent. No growth occurred in lysozyme broth. Acid was produced from adonitol, L-arabinose, D-cellobiose, dextrin, dextrose, D-fructose, D-galactose, glycerol, i-inositol, inulin, lactose, maltose, D-mannitol, D-mannose, melibiose, α-methyl-D-glucoside, α-methyl-D-mannoside, raffinose, rhamnose, D-ribose, salicin, D-sorbitol, starch, sucrose, trehalose, and D-xylose. No acid was produced from dulcitol, i-erythritol, D-melezitose and L-sorbose. Citrate, malate, succinate, oxalate, lactate, acetate, pyruvate and formate were utilized; benzoate, propionate, tartrate and mucate were not.

On the C-2 carbon utilization medium of Nonomura and Ohara, A. parvosata utilized adonitol, L-arabinose, D-cellobiose, dextrose, D-fructose, D-galactose, i-inositol, D-mannitol, raffinose, rhamnose, salicin, sucrose, and D-xylose as sole carbon sources. Inulin was poorly utilized. The vitamins in this medium were not essential for the growth of A. parvosata, but they did enhance the growth of the substrate mycelium and pigment production.

A. parvosata was resistant to disks containing rifampin (5 μg) and penicillin (10 U).

Description of A. parvosata on Various Media.

All cultures were incubated at 28°C and observed at intervals up to 21 days. The color descriptions of the culture were chosen by comparing the culture with color chips from the ISCC-NBS Centroid Color Charts, A Mycological Colour Chart (Commonwealth Mycological Institute, Kew, Surrey, & British Mycological Society, 1970), Methuen Handbook of Colour (Eyre Methuen, London, 1981), or Munsell Book of Color (Neighboring Hues Edition, Matte Finish Collection, Munsell Color Co., Inc., Baltimore, 1973).

Yeast extract-malt extract agar (ISP 2): Growth excellent; substrate mycelium violet red to dark reddish brown; aerial mycelium none to sparse, white, sterile; soluble pigment dark reddish orange to strong reddish brown, affected by pH change.

Oatmeal agar (ISP 3): Growth good; substrate mycelium dark reddish brown (Methuen 10 E6 violet brown) to violet red (Munsell 10 RP 6/6); aerial mycelium sparse, white, sterile; soluble pigment pale reddish brown to violet red, affected by pH change.

Oatmeal agar supplemented with 0.5 mg/L thiamine hydrochloride and 0.25 mg/L biotin: growth good; substrate mycelium dark reddish brown (Methuen 9 D6 reddish brown) to violet red (Munsell 10 RP 6/6); aerial mycelium none to sparse, white; spores sparse and variably present; soluble pigment reddish brown to violet red, affected by pH change.

Inorganic salts-starch agar (ISP 4): Growth fair to good; substrate mycelium yellow-brown to light orange; aerial mycelium, none; soluble pigment pale orange, not affected by pH.

Modified thin inorganic salts-starch agar: Growth good; substrate mycelium yellow-brown to light orange; aerial mycelium none to sparse, white; spores none to sparse; no soluble pigment.

Glycerol-asparagine agar (ISP 5): Growth fair to good; substrate mycelium yellow-brown to reddish brown (ISCC-NBS 43. moderate reddish brown); aerial mycelium moderate white bloom, sterile; pale yellow-brown soluble pigment variably present.

Gauze's mineral agar I: Growth fair to good; substrate mycelium yellow-brown; aerial mycelium none to sparse, white, sterile; no soluble pigment.

Gauze's organic agar 2: Growth fair; substrate mycelium reddish orange (ISCC-NBS 35. strong reddish orange); aerial mycelium none; no soluble pigment.

Synthetic agar: Growth poor to fair; substrate mycelium orange (ISCC-NBS 48. vivid orange or 50. strong orange); aerial mycelium none; no soluble pigment.

C-2 agar with dextrose: Growth good; substrate mycelium violet red to purple (ISCC-NBS 243. very dark reddish purple); aerial mycelium moderate, white to pale pink, sterile; soluble pigment pale lavender to purplish red (ISCC-NBS 262. grayish purplish red), not affected by pH.

Peptone-yeast extract-iron agar (ISP 6): Growth fair; substrate mycelium yellow-brown; aerial mycelium none; no soluble pigment.

Glucose-yeast extract agar: Growth fair to good; substrate mycelium yellow-brown to brownish orange (ISCC-NBS 54); aerial mycelium none; no soluble pigment.

A. parvosata sporulates very poorly, or not at all, on most media. It sometimes produces a moderate amount of white to pink aerial mycelium bearing numerous spores on inorganic salts-starch agar following six to eight weeks incubation at 28°C. A. parvosata sporulates more consistently on modified thin inorganic salts-starch agar, but the required incubation period is still five to seven weeks. Following five to seven weeks incubation, the aerial mycelium on this medium is pink and bears numerous spores.

On the basis of its morphological and chemotaxonomic characteristics, A. parvosata was placed in the genus Actinomadura. The description of A. parvosata was compared with the descriptions of the Actinomadura species listed on the Approved Lists of Bacterial Names or found in the patent literature. The type cultures of those species having a red, reddish brown or violet red substrate mycelium with pink aerial mycelium (A. luzonensis, A. roseoviolacea, A. roseola, A. rubra, A. salmonea, A. vinacea) were grown on a series of fifteen

plate media used in the morphological characterization of *A. parvosata*. In direct comparisons of morphology and color, these cultures all differed significantly with *A. parvosata*. The morphological observations and biochemical data from the taxonomic literature permitted easy differentiation of *A. luzonensis*, *A. roseoviolacea*, *A. roseola*, *A. rubra*, *A. salmonea*, and *A. vinacea* from *A. parvosata*.

*A. roseola* and *A. vinacea* both produce spore chains which are straight, hooked or slightly wavy. The spore surface of these two species is warty (Preobrazhenskaya, T.P., et al., "The Biology of the Actinomycetes and Related Organisms" 12:30-38, 1977). On oatmeal agar (ISP 3) *A. rubra* produces a bright reddish orange substrate mycelium and bright reddish orange soluble pigment while on glucose-yeast extract agar, it produces a dark reddish brown substrate mycelium with a bright reddish orange to scarlet soluble pigment. In addition, *A. rubra* does not produce allantoinase or urease (Athalye, M., et al., *Int. J. Syst. Bacteriol.* 35:86-98, 1985). *A. luzonensis* produces only pale orange yellow to yellow brown soluble pigments and does not utilize sucrose, raffinose or salicin as sole carbon sources (Tomita, K., et al., *J. Antibiotics* 33:1098-1102, 1980). *A. roseoviolacea* produces spore chains which are predominately tightly closed spirals that form pseudosporangia. On peptone yeast extract-iron agar, *A. roseoviolacea* produces a reddish black to black substrate mycelium, no aerial mycelium, and a greyish-reddish brown soluble pigment. *A. roseoviolacea* is resistant to lysozyme and does not degrade adenine or elastin (Goodfellow, M., et al., *J. Gen. Microbiol.* 112:95-111 1979). It also does not grow in the presence of 0.4% adenine or produce allantoinase or urease (Athalye, M. et al., *Int. J. Syst. Bacteriology* 35:86-98, 1985).

Of the cultures compared directly with A. parvosata, the spore chains of A. salmonea most closely resemble those found in A. parvosata. The spore surface of A. salmonea, however, is warty (Preobrazhenskaya, T.P., et al., "The Biology of the Actinomycetes and Related Organisms. 12:30-38, 1977). On synthetic agar, A. salmonea grows poorly and the substrate mycelium is translucent to pale yellow brown with no aerial mycelium or soluble pigment present. On Gauze's organic agar 2, the growth of A. salmonea is good to excellent with a yellow brown substrate mycelium, no aerial mycelium and a pale yellow brown soluble pigment. In addition, A. salmonea neither produces allantoinase or urease nor degrades ribonucleic acid or L-tyrosine (Athalye, M., et al., Int. J. Syst. Bacteriol. 35:86-98, 1985).

Efforts were made to obtain A. carminata for comparative taxonomic purposes. However, no culture was made available to us. Patent literature indicates that A. carminata forms spore chains arranged monopodially along the hyphae that, more often than not, are twisted in a tightly closed spiral, doughnut, or dense ball; however, they may sometimes remain straight. The number of spores per chain is from 6 to 14. The spores are round and smooth-walled. On yeast extract-malt extract agar (ISP 2) A. carminata produces a brownish violet substrate mycelium, a well-developed, fluffy, pink aerial mycelium, and no soluble pigment in the agar (Gauze, G.F. et al. Canadian Patent No. 995608, issued August 4, 1976, Anthracyclic antibiotic from Actinomadura carminata). Taxonomic literature indicates that the spore chains of A. carminata are predominately tightly closed spirals which are embedded in slime to form pseudosporangia (Meyer, J., Z. Allg. Mikrobiol. 19:37-44, 1979). On Gauze's mineral agar I, A. carminata grows poorly producing a colorless or pale reddish substrate mycelium, no or scant aerial

mycelium and no soluble pigment. On Gauze's organic agar 2 the substrate mycelium is brownish violet to blackish violet with no aerial mycelium and no soluble pigment. On oatmeal agar, growth is good with a pale lilac to reddish lilac to red violet substrate mycelium, a well-developed pink aerial mycelium and a weak soluble pigment (Preobrazhenskaya, T.P., et al., "The Biology of the Actinomycetes and Related Organisms" 12:30-38, 1977). In contrast, the predominant spore chain of *A. parvosata* is one in which the upper half to two-thirds of the spore chain forms spirals of two to six turns; these spirals may be open or closed and regularly or irregularly coiled. Pseudosporangia are seen infrequently. Spore chains containing greater than 30 spores are common in *A. parvosata*. Thus *A. parvosata* is readily distinguishable from *A. carminata* on the basis of spore chain morphology and length, as well as pigmentation and growth characteristics on various media.

Strain *A. parvosata* is thus regarded as a new species of the genus *Actinomadura* for which we propose the name *Actinomadura parvosata* (*parvus* L. adj., small in number; *satus* L. adj., seeded) referring to the few, if any, spores produced on media normally used to characterize actinomycetes.

## The Antibiotic Complex

The AAJ-271 complex refers to the mixture of the individual glycopeptide antibiotic components or biologically functional derivatives produced by the culturing or fermentation of *Actinomadura parvosata*, or an active mutant or derivative thereof. As known in the art by those familiar with antibiotic fermentation processes, the ratios of the individual or factor antibiotics and their presence in the AAJ-271 complex will vary depending upon the conditions of the fermentation process. The term

"biologically functional derivatives" or "derivatives" is used herein to include derivative compounds of components prepared chemically or biologically without adversely affecting activity, such as compounds resulting from hydrolysis of the AAJ-271 complex or its components. Examples of such derivatives of glycopeptide antibiotics can be found in U.S. Patents 4,497,802 of Debono issued February 5, 1985; 4,552,701 of Nagarajan et al. issued November 12, 1985; or 4,479,897 of Hunt et al. issued October 30, 1984. The term "active mutant or derivative" is used herein to include a microorganism which is morphologically or taxonomically similar to ATCC 53463 and which produces components of the AAJ-271 complex upon culturing in appropriate media. Such mutants and derivatives can be found in nature or can be prepared in the laboratory such as by recombinant DNA techniques or by mutagenic techniques including chemical mutagenesis or irradiation.

Typically, the AAJ-271 complex contains a mixture of AAJ-271A, AAJ-271$B_1$, AAJ-271$B_2$, AAJ-271$C_1$, AAJ-271$C_2$, AAJ-271$C_3$, AAJ-271$C_4$, and AAJ-271X, plus other unidentified components.

The AAJ-271A antibiotic has the following characteristics as indicated by fast atom bombardment mass spectrometry (hereinafter FAB MS). It has an apparent molecular weight of 1702. Fragment ions indicate the presence of a hexose and a glycolipid of molecular weight 347. The complex has a pI of 3.8. The glycolipid corresponds to a N-acyl-glucuronic acid moiety with a $C_{10}$ acyl straight chain. The extinction coefficient at $\lambda max$ =280nm of a 1% solution, designated as $E_{1cm}$, is 35 (1:1 $CH_3CN:H_2O$). Factor A has a melting point of over 300°C.

The AAJ-271$B_1$ antibiotic has the following characteristics as indicated by FAB MS. The apparent molecular weight is 1716. Fragment ions indicate the presence of a hexose and a glycolipid of molecular weight 361, corresponding to an N-acyl glucuronic acid with a $C_{11}$ fatty acid branched chain. $E^{1\%}$ = 47 (1:1 $CH_3CN:H_2O$). The melting point is over 300°C.

The AAJ-271$B_2$ antibiotic has the following characteristics as indicated by FAB MS. The apparent molecular weight is 1716. Fragment ions indicate the presence of hexose and a glycolipid of molecular weight 361 corresponding to an N-acyl glucuronic acid with a $C_{11}$ acyl straight chain. $E^{1\%}$ =49 (1:1 $CH_3CN:H_2O$). The melting point is over 300°C.

The AAJ-271$C_1$, AAJ-271$C_2$, AAJ$C_3$, AAJ-271$C_4$, and AAJ-271X antibiotics are represented by the following general structural formula (I):

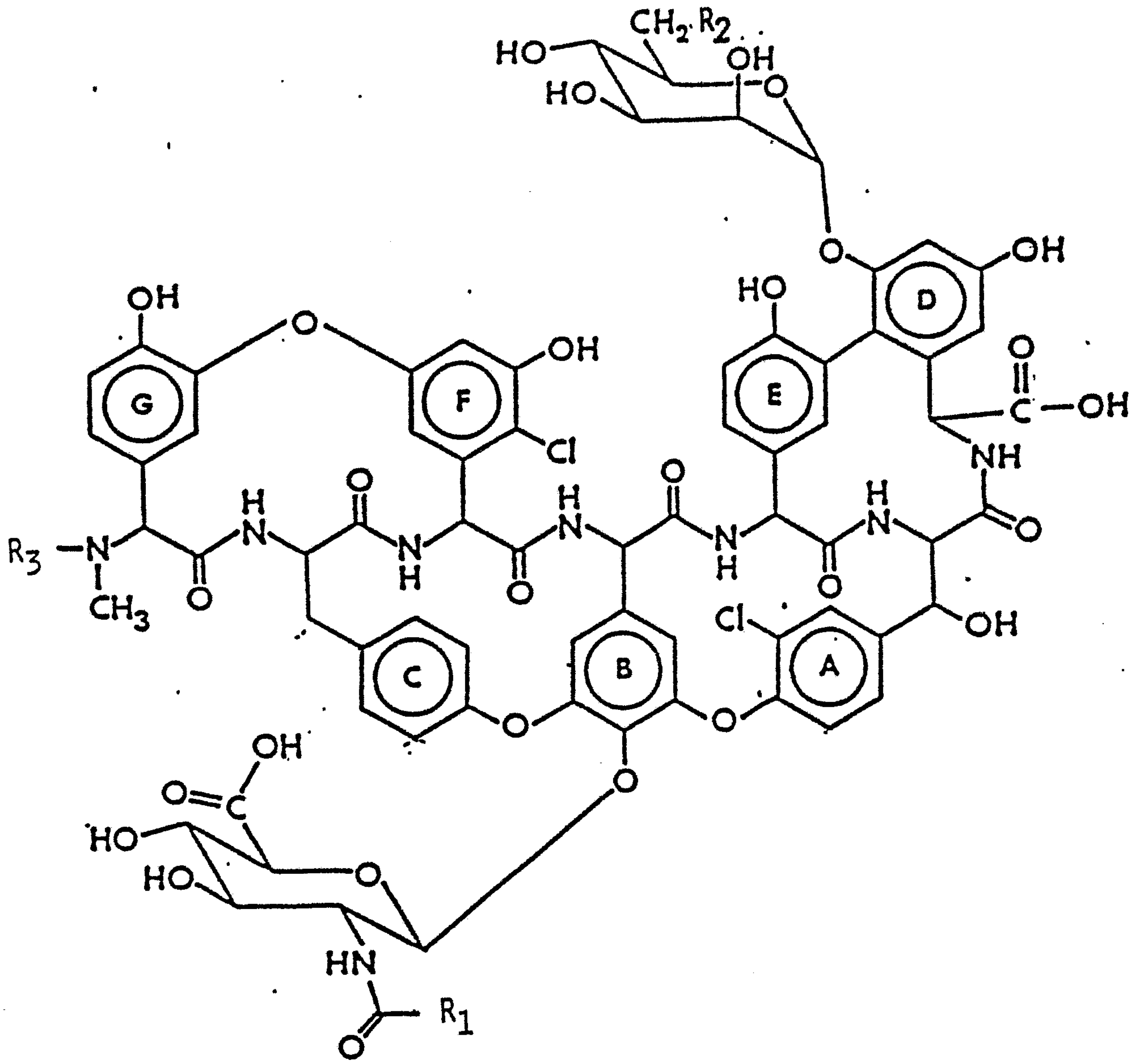

(I)

wherein $R_1$ is $C_{11}$ alkyl, $R_2$ is OH or $O\overset{O}{\overset{\|}{C}}CH_3$, and $R_3$ is H or $CH_3$, or biologically functional derivatives thereof. Each of the above identified components has a melting point of over 300°C.

For factor $C_1$, $R_1$ is 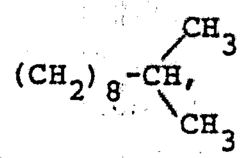

$R_2$ is OH, and $R_3$ is H. This is the major component of the AAJ-271 complex and has a determined molecular weight of 1730.496 (calculated molecular weight 1730.503) and a formula of $C_{83}H_{88}Cl_2N_8O_{29}$.

For factor $C_2$, $R_1$ is $(CH_2)_{10}CH_3$, $R_2$ is OH, and $R_3$ is H.
This component has an apparent molecular weight of 1730 and differs from AAJ-271$C_1$ only in the lack of branching in the acyl substituent on the glycolipid moiety.

For factor $C_3$, $R_1$ is 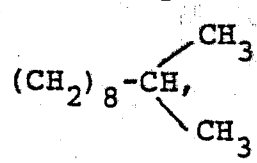

$R_2$ is $O\overset{O}{\overset{\|}{C}}CH_3$, and $R_3$ is H. The apparent molecular weight is 1772. This component is the O-acetyl derivative of AAJ-271$C_1$ at the C-6 position of the mannose.

For factor $C_4$, $R_1$ is $(CH_2)_{10}$ $CH_3$, $R_2$ is $O\overset{O}{\overset{\|}{C}}CH_3$, and $R_3$ is H. The apparent molecular weight is 1772. This component is the O-acetyl derivative of AAJ-271$C_2$ at the C-6 position of the mannose.

For factor X, $R_1$ is a $C_{11}$ alkyl group, $R_2$ is OH, and $R_3$ is $CH_3$. The nature of $R_1$, straight or branched, has not been determined. The apparent molecular weight is 1744.

Other minor factors may also be present in the complex having a similar structure wherein the length of

the alkyl chain of $R_1$ varies. $R_1$ can be a branched or straight chained alkyl of from one to fifteen carbon atoms.

The AAJ-271 complex can be prepared by cultivating <u>Actinomadura</u> <u>parvosata</u> having the characteristics of ATCC 53463, or an active mutant or derivative thereof. By active mutant or derivative thereof is meant an organism having substantially the same taxonomic characteristics as <u>A. parvosata</u> ATCC 53463 and being capable of producing the AAJ-271 antibiotic upon fermentation substantially as described herein. A fresh inolculum is grown on an agar slant using medium as previously described. The fermentation may be carried out in Erlenmeyer flasks or in labortory or industrial fermentors of various capacities. Fermentor inoculum is prepared by transferring the contents of an agar slant into an aspirator bottle containing a preferred medium (13H) composed of 1.5 g of starch, 0.5 g of sucrose, 0.5 g of dextrose, 0.75 g of soy peptone, 0.5 g of corn steep liquor, 0.15 g of $K_2HPO_4$, 0.05 g of NaCl, 0.15 g of $CaCO_3$, 5 ml of mineral "S" solution, and 0.1 ml of antifoam at pH 7.0. Mineral "S" solution consists of 0.28% $ZnSO_4.7H_2O$, 0.27% $Fe(NH_4)_2HC_6H_5O_7$, 0.125% $CuSO_4.5H_2O$, 0.1% $MnSO_4.H_2O$, 0.01%, $CoCl_2.6H_2O$, 0.01% $Na_2B_4O_7.H_2O$ and 0.005% $Na_2MoO_4.2H_2O$. All percentages are calculated on a weight/volume basis. The inoculated aspirator is incubated at about 25°-30°C for several days under continuous agitation. The contents of the aspirator can then be transferred to a larger seed fermentator containing 13H medium and incubated as above at an aeration rate of about 0.4 to 0.5 VVM.

When tank fermentation is used two or more seed stages for production of inoculum may be required. The production medium E-1 preferred for use for the final fermentation step contains the following: 20g of glucose,

1g of yeast extract, 1g of $CaCO_3$, 0.001g of $CoCl_2$, 10g of HySoy (a protein digest of Sheffield Chemical Co., of Union, New Jersey, U.S.A.), and 1 ml of antifoam at a pH of 7. All weights are grams per 1 liter of tap water. The temperature of incubation, aeration rate, and agitation rate recommended are about 25°-30°C, about 0.3 VVM, and about 100-150 RPM respectively. Any of the common methods known in the art can be employed in harvesting the fermentors or tanks to yield the AAJ-271 complex.

The AAJ-271 factors are prepared by isolation and chromatographic separation from the AAJ-271 complex. The clarified fermentation broth is chilled, optionally purified on an anion exchange resin, and treated with a hydrophobic interaction type of non-ionic resin such as HP-20 available from Mitsubishi of Tokyo, Japan, and eluted with 60% acetonitrile in water. The solution is concentrated to remove the acetonitrile and then aliquots are subjected to an affinity chromatography process as described in European Patent Application 132117 published January 23, 1985. In this process the impure antibiotic is contacted with a solid carrier matrix to which immobilized ligands have been attached. The ligands are selected from D-alanyl-D-alanine and X-D-alanyl-D-alanine wherein X is an amino acid radical. A sorption complex is formed between the antibiotic and the immobilizing ligand. The impurities are then removed from the solid carrier matrix and the sorption complex attached to it. The sorption complex between the antibiotic and immobilizing ligand is dissociated by employing a basic buffer solution in the presence of a polar organic solvent. Preferably the solid carrier matrix to which the immobilized ligands are then attached is Affi-Gel 10 of Bio-Rad Laboratories, Richmond, California, U.S.A. which is a N-hydroxysuccinimide ester of a derivatized crosslinked agarose gel bead containing a neutral 10 or 15

atom spacer arm. The gel is washed with a pH 7 buffer and eluted with 0.1M ammonium hydroxide in 50% acetonitrile. This basic treatment saponified the less biologically active $C_3$ and $C_4$ components which contain the O-acetyl group to the more biologically active $C_1$ and $C_2$ derivatives. Thus the treatment with base is a desirable method for preparation of derivatives. The resulting extract is concentrated. The extract is lyophilized and chromatographed using high pressure liquid chromatography on a column of reversed phase packing such as Vydac C18 (15-20 $\mu$m particle size) wide pore or Whatman ODS-3 using a gradient of acetonitrile in 0.01M pH 6.0 phosphate buffer. Fractions containing the desired factors are collected and desalted on a column of HP-20.

## Biological Activity Data

The *in vitro* minimal inhibitory concentration (MIC) of the AAJ-271 complex and its factors, and vancomycin were determined for a number of microorganisms using the standard microtiter assay procedures. The results are shown in the following tables, each of which represents a different production run of the complex.

Table I - Antimicrobial Activity

| Test Organism | MIC in µg/ml | | | | | |
|---|---|---|---|---|---|---|
| | AAJ 271 Complex | AAJ 271 $C_1$ | AAJ 271 $C_2$ | AAJ 271 $C_3$ | AAJ 271 $C_4$ | Vancomycin |
| Staph. aureus HH127 | 3.1 | 0.8 | 0.8 | 1.6 | 3.1 | 1.6 |
| Staph. aureus 910 | 1.6 | 0.4 | 0.8 | 1.6 | 3.1 | 1.6 |
| Staph. aureus 209P | 0.8 | 0.4 | 0.4 | 0.4 | 1.6 | 1.6 |
| Staph. aureus 675 | 3.1 | 0.8 | 1.6 | 3.1 | 6.3 | 1.6 |
| Staph. aureus 2620 | 1.6 | 0.8 | 0.8 | 3.1 | 3.1 | 1.6 |
| Staph. epidermidis 2479 | 12.5 | 6.3 | 12.5 | 25.0 | 25.0 | 1.6 |
| Strep. faecalis 34358 | 0.1 | 0.2 | 0.4 | 0.1 | 0.4 | 1.6 |
| Strep. faecalis 657 | 0 .10 | 0.20 | 0.20 | ≤0.05 | 0.40 | 1.6 |
| Strep. pyogenes (Group A) | ≤0.05 | ≤0.05 | ≤0.05 | ≤0.05 | ≤0.40 | 0.8 |
| Staph. epidermidis 2256 | 25.0 | 25.0 | 50.0 | 50.0 | >50.0 | 1.6 |
| Staph. saprophyticus 2657 | 6.3 | 0.4 | 0.8 | 6.3 | 12.5 | 1.6 |
| Staph. epidermidis 2588 | 6.3 | 12.5 | 12.5 | 6.3 | 12.5 | 1.6 |
| Staph. haemolyticus 651 | 25.0 | 25.0 | 50.0 | 25.0 | 25.0 | 3.1 |
| Staph. epidermidis 2265 | 25.0 | 25.0 | 25.0 | 50.0 | 50.0 | 1.6 |

Table II - Antimicrobial Activity

| Test Organism | MIC in μg/ml AAJ 271 A | AAJ 271 $B_1$ | AAJ 271 $B_2$ | Vancomycin |
|---|---|---|---|---|
| Staph. aureus HH127 | 3.1 | 1.6 | 6.3 | 3.1 |
| Staph. aureus 675 | 6.3 | 3.1 | 6.3 | 3.1 |
| Staph. aureus 2620 | 3.1 | 1.6 | 6.3 | 3.1 |
| Staph. epidermidis 2479 | 12.5 | 50.0 | 50.0 | 6.3 |
| Staph. epidermidis 2265 | 100.0 | 50.0 | 25.0 | 6.3 |
| Staph. haemolyticus 651 | 50.0 | 50.0 | 50.0 | 3.1 |
| Strep. faecalis 34358 | 3.1 | 1.6 | 3.1 | 6.3 |
| Strep. faecalis 657 | 3.1 | 3.1 | 3.1 | 3.1 |

The *in vitro* minimal bactericidal concentration (MBC) of the AAJ-271 $C_1$, $C_2$ and $C_3$ factors and vancomycin were determined for a number of microorganisms using standard assay procedures. The results are shown in Table III.

Table IIIA - Antimicrobial Activity

| Test Organism | MBC in μg/ml AAJ 271 $C_1$ | AAJ 271 $C_2$ | AAJ 271 $C_3$ | Vanco-mycin |
|---|---|---|---|---|
| Staph. aureus HH127 | 0.4 | 0.8 | 3.1 | 3.1 |
| Staph. aureus 910 | 0.4 | 0.8 | 3.1 | 1.6 |
| Staph. aureus 209P | 0.2 | 0.2 | 0.8 | 1.6 |
| Staph. aureus 209P-vancomycin resistant mutant | 50.0 | 100 | 100 | >100 |
| Staph. aureus 675 | 0.8 | 1.6 | 6.3 | 3.1 |
| Staph. aureus 2620 | 0.4 | 0.8 | 12.5 | 1.6 |
| Staph. epidermidis 2479 | 6.3 | 25.0 | 25.0 | 3.1 |
| Staph. haemolyticus 651 | 25.0 | 12.5 | 25.0 | 3.1 |
| Staph. epidermidis 2265 | 25.0 | 12.5 | 50.0 | 3.1 |
| Strep. faecalis 657 | 0.8 | 0.8 | 0.4 | >100 |
| Staph. epidermidis 2256 | 25.0 | 12.5 | 25.0 | 3.1 |
| Staph. saprophyticus 2657 | 1.6 | 3.1 | 12.5 | 3.1 |
| Staph. epidermidis 2588 | 6.3 | 6.3 | 6.3 | 3.1 |
| Staph. epidermidis 2261 | 6.3 | 12.5 | 12.5 | 3.1 |

Table IIIB - Antimicrobial Activity

| Test Organism | MBC in μg/ml AAJ 271 A | AAJ 271 $B_1$ | AAJ 271 $B_2$ | Vancomycin |
|---|---|---|---|---|
| Staph. aureus HH127 | 3.1 | 1.6 | 6.3 | 3.1 |
| Staph. aureus 675 | 12.5 | 6.3 | 12.5 | 6.3 |
| Staph. aureus 2620 | 3.1 | 3.1 | 6.3 | 3.1 |
| Staph. epidermidis 2479 | 12.5 | 100.0 | 50.0 | 6.3 |
| Staph. epidermidis 2265 | 100.0 | 50.0 | 50.0 | 6.3 |
| Staph. haemolyticus 651 | 100.0 | 50.0 | 50.0 | 12.5 |
| Strep. faecalis 34358 | 3.1 | 1.6 | 3.1 | 12.5 |
| Strep. faecalis 657 | 6.3 | 3.1 | 3.1 | 6.3 |

The *in vivo* activities of a mixture of AAJ-271$C_1$ and AAJ-271$C_2$ and the activity of vancomycin, measured as $ED_{50}$, mg/kg, was demonstrated in mice by standard methods against infection with several microorganisms by treatment with the antibiotic one hour post infections. The data are summarized in Table IV.

Table IV - Mouse Protection Assay

| Test Organism | AAJ-271$C_1C_2$ $ED_{50}$ | Vancomycin $ED_{50}$ |
|---|---|---|
| Staph. aureus HH127 (Dosage as No. of $LD_{50}$'s=61) | 5 | 2.8 |
| Staph. haemolyticus 651 (Dosage as No. of $LD_{50}$'s=9) | 10.8 | 2.2 |
| Strep faecalis 34358 (Dosage as No. of $LD_{50}$'s=3.2) | 4.1 | 1.3 |

The antibiotic compounds of the present invention including the AAJ-271 complex and its major known components A, $B_1$, $B_2$, $C_1$, $C_2$, $C_3$, and $C_4$ and mixtures thereof, exhibit antibacterial activity. The invention includes within its scope antibacterial compositions containing the AAJ-271 complex or one or more of the AAJ-271 components and an acceptable carrier.

The antibiotic compounds of the present invention also have a very long serum half-life. The compounds of the present invention have a serum half-life of 12 hours compared to 0.3 hour for vancomycin. This data was obtained in strain CD-1 mice using standard assay procedures. Antibiotic activity in blood serum was evaluated by disk agar diffusion with penicillin assay agar that was seeded with a Bacillus subtilis ATCC 6633 spore suspension as the indicator. Serum samples were evaluated against standards diluted in mouse serum and urines were evaluated against standards prepared in 1% pH 6 phosphate buffer. Assays were incubated at 28°C. Analysis of urines for concentration of AAJ-271$C_1$ showed a slow removal of compound from the body. The long half-life of the compounds of the present invention indicate usefulness prophylactically as an aid in prevention of infection.

The invention includes within its scope pharmaceutical compositions containing the AAJ-271 complex or one or more of the AAJ-271 components and a pharmaceutically acceptable carrier. The compositions may also contain other active antibacterial agents. The compositions are made up in any pharmaceutical form appropriate for the route of administration desired. Such compositions are exemplified by solid compositions for oral administration, such as tablets, capsules, pills, powders, and granules; liquid compositions for oral administration such as solutions, suspensions, emulsions,

syrups, and elixirs; and preparations for parenteral administration such as sterile solutions, suspensions, emulsions, creams, gels, or suaves.

For use as an antibacterial agent, the compositions are administered so that the concentration of the active ingredient is greater than the minimum inhibitory concentration for the particular organism treated.

## Growth Promotant Activity

The growth promotant activities of the AAJ-271 complex and components $C_1$, $C_2$ and $C_3$ were determined in a swine *in vitro* model to predict utility in monogastric animals, such as swine and poultry; and in a rumen *in vitro* model to predict utility in beef, dairy, and sheep production.

### Swine *in vitro* model

A Yorkshire barrow is surgically prepared either with an ileal cannula, which is placed 150 mm from the ileo-cecocolic junction, or a cecal cannula, which is placed midway between the apex and origin of the cecum. The animal is fed 4 times daily to restrict intake to 4.5% of body weight in a 30 kg animal or 2.5% of body weight in a 100 kg animal. The swine grower ration is:

| | (% w/w) |
|---|---|
| Medium ground shelled corn | 70.60 |
| Soybean meal, 44% | 22.00 |
| Dehydrated alfalfa meal, 17% | 4.50 |
| Calcium propionate | 0.15 |
| Vitamin/mineral premix | 2.75 |

Sampling of the material, via cannula, begins 150-180 minutes following the first morning feeding and continues any time from 30-120 minutes thereafter, depending on the quantity of material needed. The sampling is maintained in crushed ice, no cooler than 5°C, and is gassed continuously with carbon dioxide. The collected material is filtered. The filtrate is the inoculum used for incubations of the test and control samples. The gassed inoculum, 2.25 ml, is placed in each of 10 gassed test tubes, each containing 0.75 ml of a nutrient solution and 0.5 mg of each test compound. Four blank control tubes, along with the test compound tubes, are incubated 4 hours at 37°C with agitation. Four more killed tubes are included which are not incubated.

The tubes are each treated with 0.60 ml of a 25% solution of metaphosphoric acid, then, stored at -4°C until analysis. Samples are thawed and centrifuged for 25 minutes at 49,000 gravity. The supernatant liquid is decanted, sampled for gas chromatography and automatic analysis. Gas chromatography is used to analyze for the fatty acids, and known enzymatic methods for the remaining components. The results are fed into a computer for finishing to give figures in which the blank control value is 100%. Virginiamycin and carbadox are used as positive controls. The resulting data are listed in Table V. The higher level of glucose and lower level of lactic acid compared to the standard correlate with increased growth and feed utilization. A higher level of lysine generally correlates with an increase in protein utilization.

Table V - Growth Promotant Activity in Swine

| Compound (ppm) | VFA* (% Control) | LYS* (% Control) | GLU* (%Control) | LAC* (% Control) |
|---|---|---|---|---|
| Virginiamycin | | | | |
| (166.67) | 6 | 182 | 169 | 45 |
| (16.67) | 11 | 182 | 169 | 45 |
| (1.67) | 43 | 133 | 158 | 43 |
| Carbadox** | | | | |
| (166.67) | 12 | 176 | 135 | 86 |
| (16.67) | 8 | 166 | 122 | 107 |
| (1.67) | 94 | 99 | 111 | 98 |
| AAJ-271 Complex | | | | |
| (16.67) | 95 | 88 | 148 | 37 |
| AAJ-271$C_1$ | | | | |
| (16.67) | 39 | 124 | 159 | 44 |
| AAJ-271$C_2$ | | | | |
| (16.67) | 63 | 94 | 154 | 41 |
| AAJ-271$C_3$ | | | | |
| (16.67) | 102 | 69 | 147 | 36 |

* VFA refers to the total of volatile fatty acids, namely acetate, propionate, isobutyrate, butyrate, isovalerate and valerate. LYS is lysine, GLU is glucose and LAC is L-lactic acid.

** Carbadox is a feed additive having growth promotant activity in swine available from Pfizer Corp. New York, New York U.S.A.

Rumen *in vitro* model

The protocol for the rumen *in vitro* model is analogous to the protocol for the swine *in vitro* model with the following modifications:

(1) A 400 kg steer is surgically prepared with a rumen cannula.

(2) The animal is fed one time a day with the following ration:

| Finished Feed | % w/w |
|---|---|
| Cottonseed hulls | 44.0 |
| Cracked corn | 22.0 |
| Alfalfa Hay 1" | 20.0 |
| Pellet Supplement* | 10.0 |
| Liquid Molasses | 4.0 |
| | 100.0 |

| *Pellet Supplement | % w/w |
|---|---|
| Soybean Oil Meal (50% protein) | 50.0 |
| Medium Ground Corn | 32.5 |
| D/Calcium Phosphate | 6.50 |
| Plain salt | 2.50 |
| Ground limestone (Thomasville) | 3.50 |
| Urea | 2.50 |
| Vitamin A & $D_2$ Premix** | 2.50 |
| | 100.00 |

| **Vitamine A & $D_2$ Premix | % w/w |
|---|---|
| Vitamin A (30,000 IU/gm) | 5.87 |
| Vitamin $D_2$ (35274 IU/gm) | 0.50 |
| Fine ground corn | 93.63 |
| | 100.00 |

(3) Manipulation of VFA production is described as the production ratio of propionate as a percentage of total VFA produced.

(4) Sampling of the material, via the cannula, is at 120 minutes post feeding.

(5) Avoparcin and monensin are used as the positive controls.

The resulting data is listed in Table VI.

Table VI - Growth Promotant Activity in Rumen

| Compound (ppm) | ACE | PRO | IBU | BUT | IVA | VAL | Total | %PR |
|---|---|---|---|---|---|---|---|---|
| | (% Control)* | | | | | | | |
| Avoparcin | | | | | | | | |
| (50.00) | 98 | 106 | 104 | 107 | 112 | 77 | 101 | 105 |
| (5.00) | 116 | 114 | 96 | 106 | 87 | 95 | 109 | 105 |
| (0.50) | 105 | 103 | 97 | 97 | 101 | 100 | 101 | 101 |
| Monensin | | | | | | | | |
| (50.00) | 94 | 146 | 67 | 67 | 85 | 73 | 95 | 154 |
| (5.00) | 97 | 115 | 75 | 85 | 102 | 108 | 98 | 117 |
| (0.50) | 108 | 99 | 91 | 108 | 123 | 108 | 106 | 93 |
| AAJ-271 Complex | | | | | | | | |
| (50.00) | 117 | 138 | 116 | 111 | 120 | 74 | 117 | 119 |
| (5.00) | 115 | 116 | 117 | 118 | 119 | 118 | 117 | 99 |
| (0.50) | 128 | 113 | 85 | 112 | 92 | 109 | 115 | 98 |
| AAJ-271 $C_1$ | | | | | | | | |
| (50.00) | 110 | 127 | 122 | 111 | 113 | 63 | 110 | 115 |
| (5.00) | 84 | 104 | 108 | 108 | 110 | 107 | 100 | 104 |
| (0.50) | 108 | 95 | 105 | 104 | 113 | 107 | 104 | 91 |
| AAJ-271 $C_2$ | | | | | | | | |
| (50.00) | 109 | 129 | 132 | 104 | 121 | 64 | 109 | 118 |
| (5.00) | 117 | 119 | 116 | 111 | 93 | 105 | 113 | 105 |
| (0.50) | 107 | 102 | 108 | 110 | 119 | 112 | 108 | 95 |
| AAJ-271 $C_3$ | | | | | | | | |
| (50.00) | 110 | 127 | 95 | 105 | 90 | 63 | 107 | 119 |
| (5.00) | 106 | 107 | 82 | 101 | 83 | 98 | 102 | 105 |
| (0.50) | 120 | 105 | 94 | 107 | 101 | 113 | 111 | 95 |

* ACE is acetate, PRO is propionate, IBU is isobutyrate, BUT is butyrate, IVA is isovalerate, VAL is valerate, TOTAL is total of preceeding volatile fatty acids, and %PR is production ratio of propionate as % of total volatile fatty acids produced.

The higher %PR compared to the standard correlates with increased growth and with increased efficiency of feed utilization. The fact that propionate is increased while butyrate is not indicates that milk production will not be adversely affected. The AAJ-271 antibiotics can be used in improving milk production (fat-correlated milk yield) in lactating ruminents. The compounds can also be used to prevent and to treat ketosis in ruminants.

Thus, the AAJ-271 antibiotics can be used to increase efficiency of feed utilization. This is manifested as decreased feed intake relative to output, i.e., growth (meat production), lactation (fat-corrected milk yield) or other output, e.g., wool.

The feed compositions of this invention comprise the normal feed rations of the meat and milk producing animals, e.g., swine, poultry, sheep and bovines supplemented by a quantity of an active ingredient selected from the group consisting of AAJ-271 complex, and its factors A, $B_1$, $B_2$, $C_1$, $C_2$, $C_3$, $C_4$, X or a mixture thereof which is effective for improving the growth rate or feed efficiency of the animals but which is not toxic or noxious to a degree that the animals will reduce ingestion of the ration. The quantity of the active ingredient will vary, as is known to the art, with factors such as the cost of the ingredient, the species and the size of animal, the relative activity of the compound of formula I or the type of feed ration used as the basal feed.

Representative feed rations for swine and poultry are as follows:

A swine ration for growing hogs of 18.1 - 45.4 kg body weight is prepared using the following formula:

| | |
|---|---|
| Corn, ground | 78.15% |
| Soybean oil meal, 44% | 17.0% |
| Meat scrps, 50% | 3.0% |
| Oyster shell flavor | 0.4% |
| Bone meal | 0.5% |
| Zinc oxide | 0.01% |
| Vitamin A, B, $B_{12}$ & D supplement | optional |

A chicken ration for broilers is prepared using the following formula:

| | |
|---|---|
| Yellow corn meal | 67.35% |
| Soybean oil meal | 24.00% |
| Menhaden fish meal | 6.00% |
| Steamed bone meal | 1.00% |
| Ground limestone | 1.00% |
| Iodized salt | 0.34% |
| 25% choline chloride | 0.13% |
| Vitamine $B_{12}$ | 0.10% |
| Manganese sulfate | 0.02% |
| Vitamin mix | 0.06% |

Swine feed from weanling to fattening or finishing rations may be supplemented. Swine eat from about 90.7 gm of ration per day (for a 11.3 kg pig) to 40.82 gm per day (for a 57,9 kg pig). Most rations are comprised of a corn base supplemented with legume silage, wheat bran, oats, barley, molasses or a protein supplement.

Poultry feeds comprise starter rations, broiler rations and laying rations. The rations are usually based on ground corn, corn meal or soybean meal. The broiler rations, often, contain high energy supplements such as added fats, proteins and vitamins. Turkey rations are similar, but comprise only a starting ration and a growing ration. Chickens or pheasants eat from 13.6 - 136.1 gm of feed per day, turkeys twice that much. Estimated intake of feed is dependent on the weight and age of the meat producing animal.

Such feed rations are uniformly mixed with the compounds of the present invention effective in enhancing feed utilization and promoting growth. The active ingredients are the AAJ-271 complex, its components A, $B_1$, $B_2$, $C_1$, $C_2$, $C_3$, $C_4$, its other bioactive components, its bioactive derivatives, or a mixture thereof. The resulting supplemented rations are then fed as to custom, which is, most often, <u>ad libitum</u>. To do this conveniently, a premix of the supplemental growth

promotant of this invention, optionally combined with or without other supplements known to this art such as an anthelmintic, a nitrogen source or an antibiotic, is prepared by the manufacturer for sale to the formulators or feed lot operators. The concentration of the active ingredients in the premix is usually from 5-75% by weight or a concentration 100-2000 times greater than that in the complete feed ration. The premix form may be liquid or solid. Premix vehicles are corn oil, cottonseed oil, molasses or distillers solubles to form a liquid premix preparation. Sucrose, lactose, corn meal, ground corn, flour, calcium carbonate or soybean meal are often used as bases for solid premix preparations. The premix composition is then mixed uniformly with whole ration which is fed to the target animal. Such premix compositions are included in the term "feed compositions" as used herein.

The concentration of the active ingredients in the complete ration is a nontoxic but active quantity chosen, for example, from a range of about 1-1000 parts of active ingredient by weight per million parts of whole feed (ppm). Advantageously, a nontoxic quantity of active ingredient is chosen from the range of 10-50 ppm.

The method of this invention comprises feeding to monogastric or ruminant, meat or milk producing animals, especially beef and dairy cattle, sheep, swine and poultry, an effective growth promoting but nontoxic quantity of an active ingredient comprising the AAJ-271 complex, its components A, $B_1$, $B_2$, $C_1$, $C_2$, $C_3$, $C_4$, its other bioactive components, its bioactive derivatives or a mixture thereof. Other monogastric animals included within the method of this invention whose digestive tract also features fermentation in a cecum or cecum-like chamber are rabbits and horses.

The supplemented feed rations, described above, are presented to the animal by methods known to the art. Ad libitum feeding in the pasture, pen, or growing shed is most convenient to increase the growth and milking rate of the animal and to increase the feed efficiency of the operation.

The AAJ-271 Aglycone and Pseudo-aglycones

This invention further comprises the aglycone, pseudo-aglycones and derivatives of the AAJ-271 antibiotics represented by the following general structural formula (II):

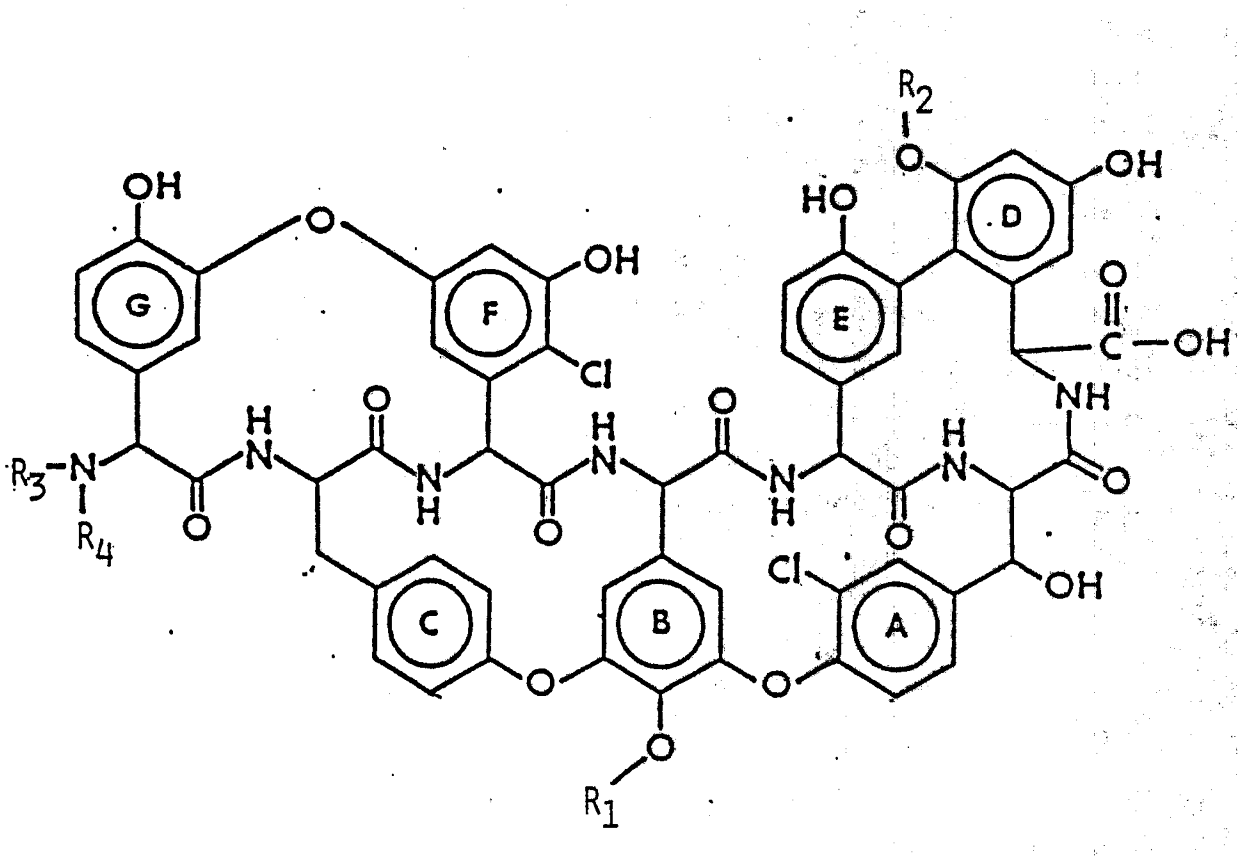

(II)

 

wherein $R_1$ is a glycolipid radical or hydrogen, $R_2$ is hydrogen or a monosaccharide radical, and $R_3$ and $R_4$, which can be same or different, are each hydrogen or methyl provided that at least one of $R_1$ and $R_2$ is hydrogen.

Preferably, the monosaccharide of $R_2$ is mannose and the glycolipid radical of $R_1$ is a hexuronic acid moiety. In particular, $R_1$ is a $C_{2-16}$ alkanoyl-aminoglucuronic acid moiety.

The AAJ-271 aglycones have the structure of formula (II) wherein $R_1$ and $R_2$ are hydrogen, $R_3$ is hydrogen or $CH_3$ and $R_4$ is $CH_3$. The AAJ-271 pseudo-aglycones have the structure of formula (II) wherein at least one of $R_1$ or $R_2$ is hydrogen, $R_3$ is hydrogen or $CH_3$, and $R_4$ is $CH_3$.

The aglycone and pseudo-alglycones of the AAJ-271 antibiotics, which include the AAJ-271 complex and its major known factors A, $B_1$, $B_2$, $C_1$, $C_2$, $C_3$, $C_4$ and X are prepared by acid hydrolysis. The hydrolysis can proceed along two alternate pathways: 1) removal of the glycolipid radical followed by removal of the monosaccharide radical; or 2) removal of the monosaccharide radical followed by removal of the glycolipid radical.

For the first pathway, acid hydrolysis of a mixture of AAJ-271 factors A, $B_1$, $B_2$, $C_1$, $C_2$, $C_3$ and $C_4$ results in a single pseudoaglycone of formula (II) wherein $R_1$ and $R_3$ are hydrogen, $R_2$ is mannosyl and $R_4$ is $CH_3$. This pseudo-aglycone is designated as AAJ-271 pseudo-aglycone since it is independent of which antibiotic factor is hydrolyzed except for factor X. Hydrolysis of AAJ-271X yields a pseudo-aglycone wherein $R_1$ is hydrogen, $R_2$ is mannosyl, and $R_3$ and $R_4$ are each $CH_3$, designated herein as the AAJ-271X pseudo-aglycone 1. Continued hydrolysis to remove the mannosyl results in the AAJ-271 aglycones. Hydrolysis of the AAJ-271 pseudo-aglycone yields an aglycone wherein $R_1$, $R_2$ and $R_3$ are hydrogen and $R_4$ is $CH_3$, designated herein aglycone 1. Hydrolysis of the AAJ-271X pseudo-aglycone yields an aglycone wherein $R_1$ and $R_2$ are hydrogen, and $R_3$ and $R_4$ are $CH_3$, designated herein aglycone 2.

Hydrolysis according to the second pathway of a mixture of AAJ-271 factors A, $B_1$, $B_2$, $C_1$, $C_2$, $C_3$, and $C_4$, involves loss of the monosaccharide radical to yield pseudo-aglycones of factors A, $B_1$, $B_2$, $C_1$, and $C_2$ having formula (II) wherein $R_1$ is a glycolipid radical, $R_2$ and $R_3$ are H, and $R_4$ is $CH_3$. The pseudo-aglycones of factors $C_1$ and $C_3$ are the same. The pseudo aglycones of factors $C_2$ and $C_4$

are the same. The compounds are designated AAJ-271 A pseudo-aglycone, AAJ-271$B_1$ pseudo-aglycone, AAJ-271$B_2$ pseudo-aglycone, AAJ-271$C_1C_3$ pseudo-aglycone, AAJ-271$C_2C_4$ pseudo-aglycone, and the like. For AAJ-271$C_3$ and AAJ-271$C_1$ pseudo-aglycone, $R_1$ is a N-acyl glucuronic acid having a $C_{12}$ acyl group with terminal iso-branching. For the AAJ-271$C_2$ and AAJ-271$C_4$ pseudo-aglycones, $R_1$ is a N-acyl glucuronic acid having a n-$C_{12}$ fatty acyl group.

Hydrolysis according to the second pathway of AAJ-271X yields a pseudo-aglycone wherein $R_1$ is a N-acyl glucuronic acid having a $C_{12}$ acyl group, $R_2$ is H, and $R_3$ and $R_4$ are methyl, designated herein as the AAJ-271X pseudo-aglycone 2.

Other minor pseudo-aglycones can be present wherein $R_1$ is a N-acyl glucuronic acid having a $C_{10-12}$ acyl group, $R_2$ is H, and $R_3$ and $R_4$ are methyl.

The AAJ-271 pseudo-aglycones of formula II have substituents as summarized in Table VII.

## Table VII - Pseudo-Aglycone Substituents

| | $R_1$ | $R_2$ | $R_3$ | $R_4$ |
|---|---|---|---|---|
| AAJ-271 Pseudo-aglycone | H | Mannosyl | H | $CH_3$ |
| AAJ-271X Pseudo-aglycone 1 | H | Mannosyl | $CH_3$ | $CH_3$ |
| AAJ-271A Pseudo-aglycone | c* | H | H | $CH_3$ |
| AAJ-271$B_1$ Pseudo-aglycone | d* | H | H | $CH_3$ |
| AAJ-271$B_2$ Pseudo-aglycone | d* | H | H | $CH_3$ |
| AAJ-271$C_1$/$C_3$ Pseudo-aglycone | a* | H | H | $CH_3$ |
| AAJ-271$C_2$/$C_4$ Pseudo-aglycone | b* | H | H | $CH_3$ |
| AAJ-271X Pseudo-aglycone 2 | e* | H | $CH_3$ | $CH_3$ |

* a is

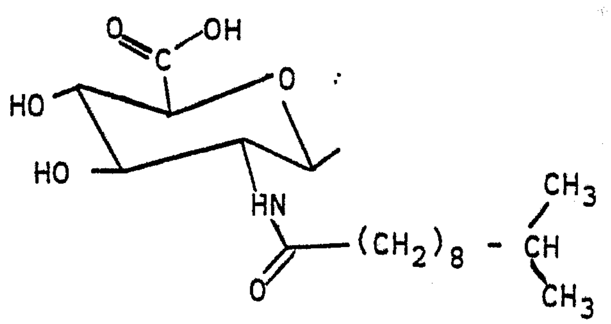

* b is

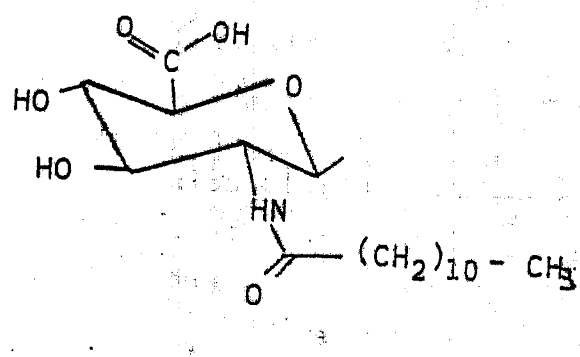

* c = a or b with a $C_{10}$ acyl group in place of the $C_{12}$ fatty acyl group.

* d = a or b with a $C_{11}$ acyl group in place of the $C_{12}$ acyl group.

* e = N-acyl glucuronic acid having $C_{12}$ acyl group of undetermined structure.

The AAJ-271 pseudo-aglycone and AAJ-271X pseudo-aglycone 1 are prepared by hydrolyzing the AAJ-271 antibiotics by the first pathway described above, in aqueous organic solvent and very dilute mineral acid at reflux until a substantial amount of the AAJ-271 pseudo-aglycone and AAJ-271X pseudo-aglycone 1 are formed. Subsequently they are isolated from the reaction mixture. Illustrative of this hydrolysis process is the treatment of AAJ-271$C_1$ in 10 percent aqueous acetonitrile with 0.001 N hydrochloric at reflux for 48 hours and the isolation of the resulting AAJ-271 pseudo-aglycone by chromatographic means.

The AAJ-271 aglycones, the AAJ-271A, $B_1$, $B_2$, $C_1/C_3$, $C_2/C_4$, and X2 pseudo-alglycones are prepared by the hydrolysis of the appropriate AAJ-271 factor compound in a polar organic solvent and dilute mineral acid at elevated temperature until a substantial amount of the hydrolysis products are formed, and subsequently isolating the individual desired products. Illustrative of this hydrolysis process is the treatment of AAJ-271$C_1$ in dimethylsulfoxide with 5 percent hydrochloric acid at 100°C for 15 minutes. The isolation of the individual AAJ-271 aglycones of formula II wherein $R_1$, $R_2$ and $R_3$ are hydrogen and $R_4$ is methyl, and the AAJ-271$C_1/C_3$ pseudo-aglycone may be accomplished by chromatographic means.

Alternatively, the AAJ-271 aglycone and the pseudo-aglycones may be prepared by the mild acid hydrolysis of the AAJ-271 complex or the individual factors followed by a chromatographic isolation of the desired compounds.

Aglycone Biological Activity Data

The *in vitro* minimal inhibitory concentration (MIC) and the *in vitro* minimal bactericidal concentration (MBC) of the AAJ-271 aglycones, pseudo-aglycone and vancomycin were determined for a number of microorganisms using the standard microtiter assay procedures. The results are shown in Tables VIII and IX.

Table VIII - Aglycone Antimicrobial Activity

| Test Organism | MIC in µg/ml | | | MBC in µg/ml | | |
|---|---|---|---|---|---|---|
| | Aglycone 1 | Aglycone 2 | Vancomycin | Aglycone 1 | Aglycone 2 | Vancomycin |
| Staph. aureus HH127 | 0.4 | 0.4 | 1.6 | 1.6 | 0.4 | 1.6 |
| Staph. aureus 910 | 0.4 | 0.4 | 1.6 | 3.1 | 0.4 | 1.6 |
| Staph. aureus 209P | 0.4 | 0.4 | 0.8 | 3.1 | 0.4 | 0.8 |
| Staph. aureus 209P vancomycin resistant mutant | 25.0 | 25.0 | >100.0 | 25.0 | 25.0 | >100.0 |
| Staph. aureus 675 | 0.8 | 0.4 | 3.1 | 6.3 | 0.4 | 3.1 |
| Staph. aureus 2620 | 0.4 | 0.8 | 1.6 | 3.1 | 0.8 | 1.6 |
| Staph. epidermidis 2479 | 0.8 | 0.8 | 3.1 | 6.3 | 0.8 | 6.3 |
| Staph. haemolyticus 651 | 3.1 | 6.3 | 3.1 | 6.3 | 6.3 | 3.1 |
| Staph. epidermidis 2265 | 0.4 | 0.8 | 3.1 | 0.4 | 0.8 | 3.1 |
| Strep. faecalis 34358 | 1.6 | 1.6 | 3.1 | 100.0 | >50.0 | 50.0 |
| Strep. faecalis 657 | 1.6 | 1.6 | 3.1 | 100.0 | 6.3 | 3.1 |
| Strep. pyogenes (Group A) | 0.8 | 1.6 | 0.8 | 0.8 | 0.8 | 0.8 |
| Staph. epidermidis 2256 | 1.6 | 0.8 | 3.1 | 1.6 | 0.8 | 3.1 |
| Staph. saprophyticus 2657 | 0.8 | 0.8 | 3.1 | 1.6 | 0.8 | 6.3 |
| Staph. epidermidis 2588 | 0.8 | 1.6 | 6.3 | 0.8 | 3.1 | 6.3 |
| Staph. epidermidis 2261 | 0.8 | 1.6 | 6.3 | 1.6 | 1.6 | 3.1 |

Table IX - Pseudo-aglycone Antimicrobial Activity

| Test Organism | MIC in µg/ml | | MBC in µg/ml | |
|---|---|---|---|---|
| | AAJ 271 Pseudo-aglycone | Vancomycin | AAJ 271 Pseudo-aglycone | Vancomycin |
| Staph. aureus HH127 | 3.1 | 3.1 | 6.3 | 3.1 |
| Staph. aureus 675 | 3.1 | 3.1 | 3.1 | 6.3 |
| Staph. aureus 2620 | 3.1 | 3.1 | 3.1 | 3.1 |
| Staph. epidermidis 2479 | 6.3 | 6.3 | 6.3 | 6.3 |
| Staph. epidermidis 2265 | 12.5 | 6.3 | 12.5 | 6.3 |
| Staph. haemolyticus 651 | 25.0 | 3.1 | 25.0 | 12.5 |
| Strep. faecalis 34358 | 3.1 | 6.3 | 3.1 | 12.5 |
| Strep. faecalis 657 | 3.1 | 3.1 | 3.1 | 6.3 |

The *in vivo* activity of AAJ-271 aglycone 1 and vancomycin, measured as $ED_{50}$, mg/kg, was demonstrated in mice against intraperitoneal s.c. infections with several organisms by treatment with the antibiotic one hour post infections. The data are summarized in Table X.

Table X - Aglycone Mouse Protection Assay

| Test Organism | Aglycone $ED_{50}$ | Vancomycin $ED_{50}$ |
|---|---|---|
| Staph. aureus HH127 (Dosage as No. of $LD_{50}$'s = 4) | 0.88 | 0.26 |
| Staph. aureus 2630 (Dosage as No. of $LD_{50}$'s = 18.7) | 6.4 | 0.78 |
| Strep. faecalis 34358 (Dosage as No. of $LD_{50}$'s = 3.5) | 5.0 | 1.25 |

The AAJ-271 aglycones and pseudo-aglycones exhibit antibacterial activity. The invention includes within its scope pharmaceutical compositions containing one or more of the AAJ-271 aglycones or pseudo-aglycones and a pharmaceutically acceptable carrier. The compositions may also contain other active antibacterial agents, and can be in any of several pharmaceutical forms as previously listed for the AAJ-271 complex. The compositions are administered so that the concentration of the active ingredient is greater than the minimum inhibitory concentration for the particular organism treated.

The following examples are illustrative of the production, isolation and purification of the antibiotics of the present invention and are not therefore to be considered in limiting the present invention as described in the claims appended hereto.

The nutrient media employed in the following examples have the compositions listed below. The yields for the fermentations were obtained by analytical HPLC by comparison to quantitated authentic samples.

Medium 13H was used for the seed growth of AAJ-271 throughout the experiments. Ingredients of medium 13H are: starch, 15 g/l; sucrose, 5 g/l; dextrose, 5 g/l; HY-soy, 7.5 g/l; corn steep liquor, 5 g/l; $K_2HPO_4$, 1.5 g/l; NaCl, 0.5 g/l; $CaCO_3$, 1.5 g/l; and Mineral "S", 5 ml/l; pH to 7.0.

Mineral "S" has the following compositions: $ZnSO_4 7H_2O$, 2.8 g/l; $Fe(NH_4)_2HC_6H_5O_7$, 2.7 g/l; $CuSO_4.5H_2O$, 0.125 g/l; $MnSO_4.H_2O$, 1.0 g/l; $CoCl_2.6H_2O$, 1.0 g/l; $Na_2B_4O_7.H_2O$, 0.9 g/l; and $Na_2MoO_4.2H_2O$, 0.5 g/l.

Medium E1 has the following composition: glucose-20 g/l; yeast extract-1 g/l; $CaCO_3$- 1g/l; $CoCl_2$-0.001 g/l; HySoy-10 g/l; pH to 7.

EXAMPLE 1

A fresh inoculum of AAJ-271 was grown on an agar slant of medium of thin Difco inorganic salts-starch agar [ISP 4]-12 g; Difco Bacto agar - 15 g; distilled water - 1 liter, supplemented with yeast extract at 2 g/l. The entire growth from that slant was used to inoculate a 4 L aspirator bottle containing 500 ml of medium 13H. The aspirator bottle was then incubated at 28°C and agitated at 250 RPM (2 in. throw) for 4 days. The entire contents of the aspirator bottle were then aseptically transferred to a 20 L seed fermentor containing 14 L of medium 13H. The temperature of incubation, aeration rate, and agitation rate were 28°C, 0.43 VVM, and 300 RPM, respectively. After 4 days, approximately 10 L of broth were transferred to a 150 L, seed fermentor containing 100 L of medium 13H. The temperature of incubation, aeration rate, and agitation rate were 28°C, 0.5 VVM, and 175 RPM, respectively. After 4 days, the entire contents of the 150 L seed fermentor were transferred to a 750 L, production fermentor containing 500 L of medium E1. The temperture of incubation, aeration rate, and agitation rate were 28°C, 0.3 VVM, and 100 RPM, respectively. At 66 hours of operation the concentrations of the AAJ-271$C_1$ and AAJ-271$C_2$ components were 41.3 and 44.5 $\mu$g/mL, respectively, and the tank was harvested.

EXAMPLE 2

A fresh inoculum of AAJ-271 was grown on an agar slant of medium and the entire growth from that slant was used to inoculate a 4 L aspirator bottle containing 500 mL of medium 13H. The aspirator bottle was then incubated at 28°C and agitated at 250 RMP (2 in. stroke) for 4 days. The entire contents of the aspirator bottle were then aseptically transferred to a 20 L, seed fermentor containing 15 L of medium 13H. The temperature of incubation, aeration rate, and agitation rate were 28°C,

0.4 VVM, and 300-400 RPM, respectively. After 4 days, approximately 5 L of broth were transferred to a 75 L, seed fermentor containing 50 L of medium 13H. The temperature of incubation, aeration rate, and agitation rate were 28° C, 0.5 VVM, and 250-300 RPM, respectively. After 4 days, the entire contents of the 75 L seed fermentor were transferred to a 750 L, production fermentor containing 500 L of medium E1. The temperature of incubation, aeration rate, and agitation rate were 28°C, 0.3 VVM, and 120 RPM, respectively. The production of the AAJ-271$C_1$, component during the course of the fermentation was as follows:

| Time (hours) | $C_1$ μg/mL |
|---|---|
| 18 | 0.9 |
| 53 | 16.2 |
| 66 | 22.8 |
| 75 | 43.0 |
| 90 | 60.3 |

EXAMPLE 3

Fifteen liters of fermentation broth of Example 2 was clarified by filtration with celite and the filtrate (11 L) passed through four liters of HP 20 resin. The column was washed with 20 liters of water and eluted with 60% acetonitrile-water. The eluate was concentrated to 3 liters *in vacuo* and passed through a 200 ml column of Affigel 10-D-Ala-D-Ala in two parts. Each column was washed with water and eluted with 0.1 N ammonium hydroxide containing 50% acetonitrile. The combined eluates were neutralized with dilute HCl and lyophylized to yield three grams of crude complex. The residue was chromatographed on a Whatman Magnum 20 column (2.2 cm ID X 50 cm) packed with 10 micron ODS-3 reversed phase support using a gradient of acetonitrile in 0.1 M phosphoric acid of pH 3.2. The components were eluted with 29% acetonitrile. Fractions which were homogeneous in each component were

pooled, diluted with six volumes of water, and desalted by adsorption onto HP-20 followed by elution with 60% acetonitrile-water to yield after lyophylization 24 mg of $C_1$ ($E^{1\%}_{1cm}$ 51.2), 6.5 mg of $C_2$ ($E^{1\%}_{1cm}$ 45.3), 8 mg of $C_3$ ($E^{1\%}_{1cm}$ 46.1), and 4 mg of $C_4$ ($E^{1\%}_{1cm}$ 45.2)., on carbohydrate analysis all the factors were found to contain mannose as the neutral carbohydrate and had isoelectric points of 3.8 suggesting the presence of a glucuronic acid moiety.

EXAMPLE 4

Fermentation broth (600 L) of Example 2 was clarified using a rotary drum filter with the aid of diatomaceous earth (Hyflo Supercell of Manville Filtration and Minerals, Denver, Colorado U.S.A.) to yield 400 L of clarified broth. To the chilled broth (4°C) was added 40 L of IRA-68 anion exchange resin (Rohm and Haas, Philadelphia, Pennsylvania) with stirring and continuous addition of concentrated HCl to maintain a pH between 6 and 8. The resin was allowed to settle and the bulk of the broth decanted. The resin was washed twice with 40 L of deionized water and batch eluted with 270 L of 1 M ammonium hydroxide which was neutralized with concentrated phosphoric acid to a pH between 6 and 8. The neutralized solution was pumped onto 2 columns packed with 8 L each of Dianion HP-20 (Mitsubishi). The columns were washed with 20 L of deionized water and eluted with 20 L of 60% acetonitrile in water. The combined eluates from the two columns were concentrated *in vacuo* to 10 L, which was treated in 1 L batches with 300 ml of Affigel D-Ala-D-Ala. The gel was washed with 1 L of 0.02 phosphate buffer, pH 7, and eluted with 600 ml of 0.1 M ammonium hydroxide in 50% acetonitrile. The combined eluates were neutralized with phosphoric acid and concentrated *in vacuo* to remove the acetonitrile. The extract at this stage

contained a total of 1.5g of the $C_1$ component and 1.4g of the $C_2$ component. All of the $C_3$ and $C_4$ components were saponified to the desired $C_1$ and $C_2$ components respectively by the base treatments used in the purification process.

One g of the solid crude mixture was separated by HPLC using an HP chemical 5.1 cm ID x 50 cm L Vydac C18 column, 15-20 micrometer particle size packing, pore size 300 angstroms, detection at 250 nm. Acetonitrile in 0.01M $KH_2PO_4$ of pH 6 was employed as the solvent at a 100 ml/min. flowrate as follows:

| Time | % $CH_3CN$ |
|---|---|
| 0 - 10 min. | 15 → 20% $CH_3CN$ |
| 10 - 27 min. | 20 → 22% $CH_3CN$ |
| 27 - 47 min. | 22 → 24% $CH_3CN$ |
| 47 - 95 min. | 24 → 25% $CH_3CN$ |

At the 1 g loading level, 5mg of Components A, 20mg of $B_1$ and 10mg of $B_2$ were isolated relatively cleanly but the $C_1/C_2$ fraction eluted as a mixture. Therefore, the $C_1/C_2$ mix from this run was combined with 3 g of the solid crude mixture for subsequent chromatographic runs.

Upon completion of the subsequent runs, fractions containing the desired factors were pooled and desalted on a column of HP-20 to yield 300mg of factor $C_1$, and 230 mg of factor $C_2$.

Analysis of the isolated components by mass spectrometry indicated the following:

AAJ-271A: Molecular weight 1702, presence of a hexose and a N-acyl-glucuronic acid with a $C_{10}$ acyl chain.

AAJ-271$B_1$: Molecular weight 1716, presence of a hexose and a N-acyl-glucuronic acid with a $C_{11}$ acyl chain.

AAJ-271$B_2$: Molecular weight 1716, presence of a hexose and a N-acyl-glucuronic acid with a $C_{11}$ acyl chain.

AAJ-271$C_1$ Determined molecular weight of 1730.496, structure of formula (I) wherein

$R_1$ is 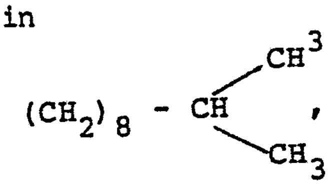 , $R_2$ is OH, and $R_3$ is H.

AAJ-271$C_2$ Molecular weight of 1730, structure of formula (I) wherein $R_1$ is $(CH_2)_{10}CH_3$, $R_2$ is OH, and $R_3$ is H.

AAJ-271$C_3$ Molecular weight of 1772, structure of formula (I) wherein $R_1$ is

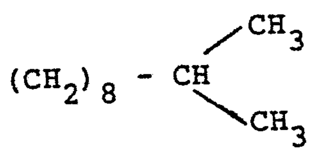

$R_2$ is $O\overset{O}{\overset{\|}{C}}CH_3$, and $R_3$ is H.

AAJ-271$C_4$ Molecular weight of 1772, structure of formula (I) wherein $R_1$ is $(CH_2)_{10}CH_3$, $R_2$ is $O\overset{O}{\overset{\|}{C}}CH_3$, and $R_3$ is H.

AJ-271X Molecular weight of 1744, structure of formula (I) wherein $R_1$ is a branched or straight chain $C_{11}$ alkyl group, $R_2$ is OH, and $R_3$ is $CH_3$.

The components can be characterized by HPLC retention times. A sample of the crude mixture was also chromatographed on a Beckman 345 system on a Brownlee ODS precolumn (4.6 mm I.D. x 30 mm. L) of 5-micrometer packing followed by a Beckman (Allex) Ultrasphere ODS column (4.6 mm I.D. x 150 mm L) of 5-micrometer packing with detection at 220 nm. The solvent system was acetonitrile and 0.01M $KH_2PO_4$ of pH 3.2 at a flow rate of 1.5 ml/min. as follows:

| | |
|---|---|
| 14% → 37% acetonitrile | 8 min. |
| 37% acetonitrile | 8 min. |
| 37% → 14% acetonitrile | 3 min. |

The following components were separated:

| Component | Retention Time, Min. ± 0.1 |
|---|---|
| AAJ-271 Mannosyl Pseudo-Aglycone | 6.17 |
| AAJ-271 Aglycone 1 | 6.81 |
| AAJ-271 Aglycone 2 | 7.06 |
| AAJ-271 A | 9.63 |
| AAJ-271 $B_1$ | 10.36 |
| AAJ-271 $B_2$ | 10.55 |
| AAJ-271 $C_1$ | 11.38 |
| AAJ-271 $C_2$ | 11.56 |
| AAJ-271 $C_3$ | 12.05 |
| AAJ-271 $C_4$ | 12.35 |

EXAMPLE 5

A sample of affinity-gel isolated AAJ-271 complex prepared as in Example 3 (105 mg) was dissolved in 5 ml of a 10% aqueous dimethylsulfoxide solution 0.3M in HCl (19 ml dimethylsulfoxide, 1 ml water, 1 ml 6N HCl) in a 25 ml round-bottomed flask fitted with condenser. The mixture was heated under nitrogen at 100-105°C for 75 min., cooled and the liquids evaporated. HPLC analysis revealed two species present in an 81:19 ratio. This material was subjected to a standard course of D-Ala-D-Ala-affinity chromatography as in Example 3. The isolated material was subjected to semi-preparative HPLC (Whatman Partisil-10, ODS packing; Magnum 20, 22.1 mm x 50 cm) using a isochratic system of 17% acetonitrile in 0.01M $KH_2PO_4$ at a pH 6.0. The two isolated components were desalted on HP-20 resin.

Two algycones were obtained:

Aglycone #1 had a molecular weight of 1211; derived empirical formula $C_{59}H_{47}Cl_2N_7O_{18}$; and an HPLC retention time of 6.81 min. using the conditions described in Example 3. Hydrolytic experiments and 500 MHz $^1H$ NMR analysis revealed this to be the aglycone of components AAJ-271 $C_1$ and $C_3$. Separate hydrolytic experiments with AAJ-271 $C_1/C_2$ and AAJ-271 $C_3/C_4$ mixtures revealed this to be the aglycone of AAJ-271 $C_2$ and $C_4$ also. $E^{1\%}_{1cm}$= 68 (1:1 $CH_3CN:H_2O$).

Aglycone #2 had a molecular weight of 1225; derived empirical formula $C_{60}H_{49}Cl_2N_7O_{18}$; and HPLC retention time of 7.06 min. using the conditions described in Example 3.

Careful integration of 500 $^1H$ NMR spectra revealed the presence of an additional methyl group on the amino-terminus. This aglycone corresponded to that of AAJ-271X. $E^{1\%}_{1cm}$ = 70 (1:1 $CH_3CN:H_2O$).

EXAMPLE 6

A mixture of AAJ-271$C_1$ and $C_2$ (2.5 g, 1.45 mmoles; 56:44 $C_1/C_2$ ratio; from preparative isolation as in Example 3) was dissolved in 1:1 acetonitrile: water (160 ml) and the solution adjusted to pH 3.0 (HCl). The mixture was heated at reflux for 20 hrs., cooled and concentrated *in vacuo*. The mixture was subjected to D-Ala-D-Ala affinity chromatography using standard procedure as in Example 3, producing 1.8 g of material after HP-20 resin desalting. This corresponded to 94% crude yield. A 250 mg. sample of this material was subjected to semi-preparative chromatography (Whatman Partisil-10 ODS-3, Magnum 20; 17% acetonitrile in 0.01 M $KH_2PO_4$/pH6.0 isochratic. After HP-20 resin desalting, 131 mg. (52% recovery) was isolated.

The resulting pseudo-aglygone had a molecular weight of 1373 and a derived empirical formula of $C_{65}H_{57}Cl_2N_7O_{23}$ $E^{1\%}_{1cm}$ = 70 (1:1 $CH_3CN:H_2O$).
This is consistant with the mannosyl aglycone of the AAJ-271 complex.

Claims for Contracting States: BE, CH, DE, FR, GB, IT, LI, LU, NL and SE

1. A biologically pure culture of Actinomadura parvosata Shearer sp. nov. ATCC 53463 or an active mutant or derivative thereof, said microorganism, mutant and derivative being capable of producing AAJ-271 antibiotics in recoverable quantity upon cultivation in aqueous nutrient medium containing assimilable sources of nitrogen and carbon.

2. An antibiotic AAJ-271 complex prepared by culturing or fermentation of Actinomadura parvosata Shearer sp. nov. ATCC 53463 microorganisms, or an active mutant or derivative thereof, until a substantial amount of the complex is produced and isolating the complex so produced.

3. An AAJ-271 antibiotic compound represented by structural formula (I) :

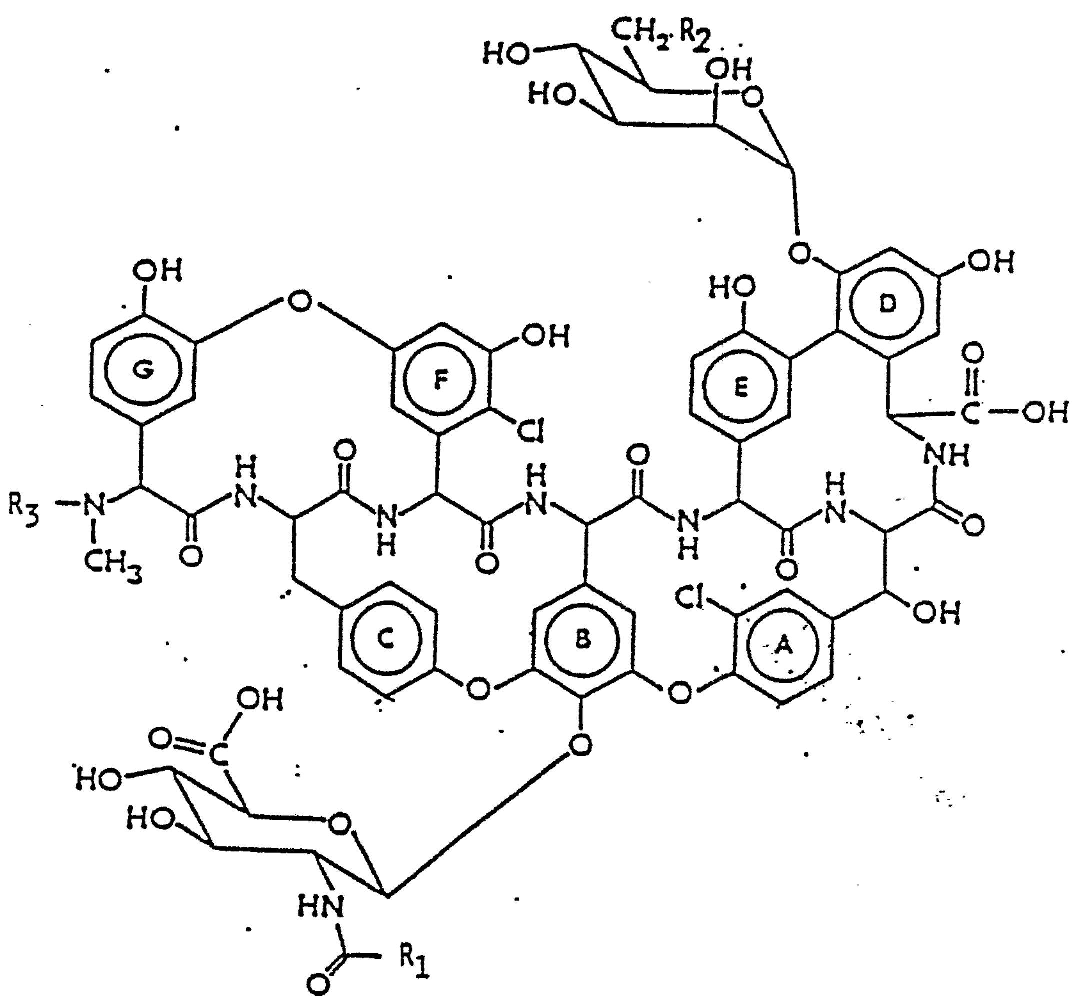

(I)

wherein $R_1$ is $C_{1-15}$alkyl, $R_2$ is OH, and $R_3$ is H or $CH_3$; or a mixture of said compounds, and biologically functional derivatives thereof.

4. The compound of Claim 3 which is :

AAJ-271$C_1$ wherein $R_1$ is 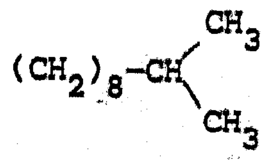, $R_2$ is OH, and $R_3$ is H;

AAJ-271$C_2$ wherein $R_1$ is $(CH_2)_{10}CH_3$, $R_2$ is OH, and $R_3$ is H;

AAJ-271$C_3$ wherein $R_1$ is 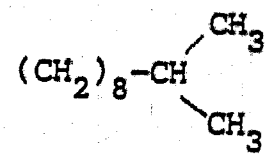, $R_2$ is $O\overset{O}{\overset{\|}{C}}CH_3$, and $R_3$ is H;

AAJ-271$C_4$ wherein $R_1$ is $(CH_2)_{10}CH_3$, $R_2$ is $O\overset{O}{\overset{\|}{C}}CH_3$, and $R_3$ is H;

AAJ-271X wherein $R_1$ is a straight or branched $C_{11}$ alkyl chain, $R_2$ is OH, and $R_3$ is $CH_3$;

AAJ-271A having a molecular weight of 1702 and containing hexose, and a N-acyl glucuronic acid moiety with a n-$C_{10}$ acyl group;

AAJ-271$B_1$ having a molecular weight of 1716 and containing a hexose and a N-acyl glucuronic acid moiety with a $C_{11}$ acyl group having terminal iso-branching; or

AAJ-271$B_2$ having a molecular weight of 1716 and containing a hexose and a N-acyl glucuronic acid moiety with a n-$C_{11}$acyl group.

5. The antibiotic AAJ-271 of Claim 3 comprising a mixture of two or more compounds AAJ-271A, AAJ-271$B_1$, AAJ-271$B_2$, AAJ-271$C_1$, AAJ-271$C_2$, AAJ-271$C_3$, AAJ-271$C_4$ or AAJ-271X or biologically functional derivatives thereof.

6. A process for the manufacturing of the antibiotic complex of Claim 2 comprising culturing _Actinomadura_ _parvosata_ Shearer sp. nov. ATCC 53463 microorganisms or mutants thereof in an aqueous nutrient medium containing assimilable sources of nitrogen and carbon under submerged aerobic conditions until a substantial amount of the complex is produced and isolating the complex so produced.

7. The process of Claim 6 further comprising the isolation by chromatographic means of AAJ-271A, $B_1$, $B_2$, $C_1$, $C_2$, $C_3$, or $C_4$ components or biologically functional derivatives thereof.

8. An antibiotic compound represented by structural formula (II)

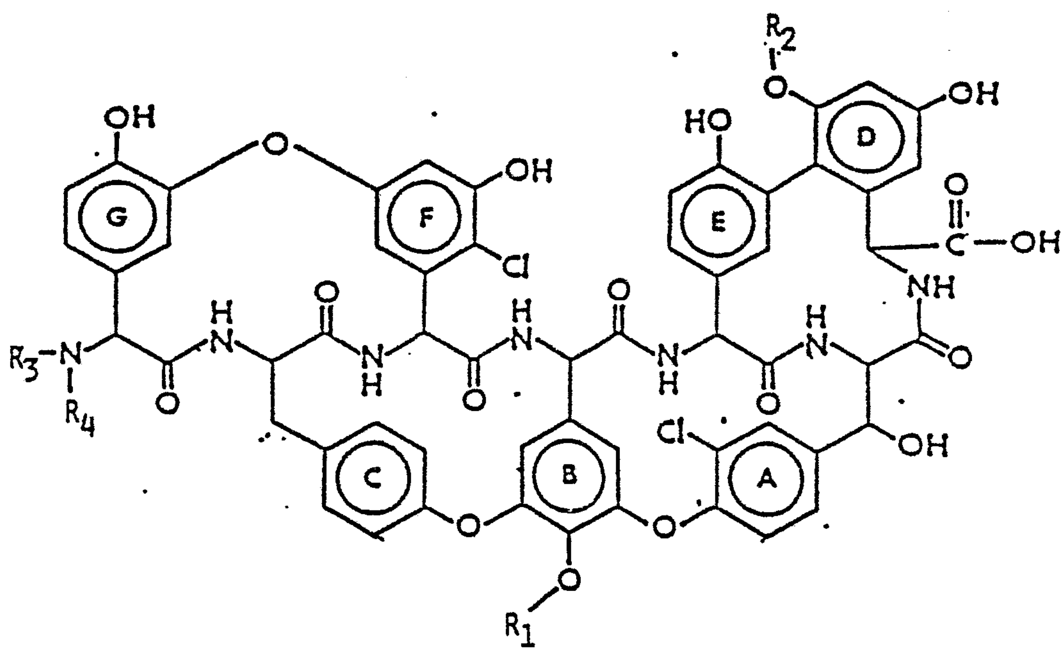

(II)

wherein $R_1$ is hydrogen or a glycolipid radical, $R_2$ is hydrogen or monosaccharide radical, $R_3$ is hydrogen or methyl, and $R_4$ is hydrogen or methyl with the proviso that at least one of $R_1$ and $R_2$ is hydrogen.

9. A compound of claim 8 which is :

AAJ-271 aglycone 1 wherein $R_1$, $R_2$ and $R_3$ are hydrogen and $R_4$ is methyl;

AAJ-271 aglycone 2 wherein $R_1$ and $R_2$ are hydrogen and $R_3$ and $R_4$ are each methyl;

AAJ-271 pseudo-aglycone wherein $R_1$ is hydrogen, $R_2$ is mannosyl, $R_3$ is hydrogen and $R_4$ is methyl;

AAJ-271X pseudo-aglycone 1 wherein $R_1$ is hydrogen, $R_2$ is mannosyl, and $R_3$ and $R_4$ are each methyl;

AAJ-271$C_1$/$C_3$ pseudo-aglycone wherein $R_1$ is N-acyl glucuronic acid with a $C_{12}$ acyl group having terminal iso-branching, $R_2$ and $R_3$ are hydrogen, and $R_4$ is methyl;

AAJ-271$C_2$/$C_4$ pseudo-aglycone wherein $R_1$ is N-acyl glucuronic acid with a n-$C_{12}$ acyl group, $R_2$ and $R_3$ are hydrogen, and $R_4$ is methyl;

AAJ-271A pseudo-aglycone wherein $R_1$ is N-acyl glucuronic acid with a $C_{10}$ acyl group, $R_2$ and $R_3$ are H, and $R_4$ is $CH_3$;

AAJ-271$B_1$ pseudo-aglycone or wherein $R_1$ is N-acyl glucuronic acid with a $C_{11}$ acyl group having terminal iso-branching, $R_2$ and $R_3$ are H, and $R_4$ is $CH_3$;

AAJ-271 $B_2$ pseudo-aglycone wherein $R_1$ is N-acyl glucuronic acid with a n-$C_{11}$acyl group, $R_2$ and $R_3$ are H, and $R_4$ is $CH_3$;

AAJ-271X pseudo-aglycone 2 wherein $R_1$ is N-acyl glucuronic acid with a $C_{12}$ acyl group, $R_2$ is H, and $R_3$ and $R_4$ are $CH_3$; or

minor pseudo aglycones wherein $R_1$ is N-acyl glucuronic acid with a $C_{10-12}$ acyl group, $R_2$ is H, and $R_3$ and $R_4$ are $CH_3$.

10. The antibiotic of Claim 9 comprising a mixture of any two or more of the glycones or pseudo-aglycones of the AAJ-271 complex or its factors or their biological functional derivatives.

11. A process for the preparation of the compounds of Claims 8 or 9 comprising the partial acid hydrolysis of one or more antibiotics selected from the group consisting of AAJ-271 complex, and AAJ-271 factors A, $B_1$, $B_2$, $C_1$, $C_2$, $C_3$, $C_4$, or X, until a substantial amount of the compounds are formed and isolating said compounds.

12. A composition comprising a pharmaceutically acceptable carrier and a product as defined in any one of Claims 2 to 5 and 8 to 10, or any mixture thereof.

13. An animal feed composition comprising an animal feed and a product as defined in any one of Claims 2 to 5 and 8 to 10, or any mixture thereof.

14. An animal feed premix composition comprising a premix vehicle and a product as defined in any one of Claims 2 to 5 and 8 to 10, or any mixture thereof.

15. The use of a compound or composition as defined in any one of Claims 2 to 5, 8 to 10 and 12 in the manufacture of a medicament having antibacterial activity.

16. A method of improving the growth rate or feed efficiency of meat or milk producing animals which comprises administering a product or composition as defined in any one of Claims 2 to 5, 8 to 10 and 12 to 14.

17. A method of increasing propionate production in the rumen or cecum of meat or milk producing animals which comprises administering a product or composition as defined in any one of Claims 2 to 5, 8 to 10 and 12 to 14.

Claims for Contracting States: AT, GR and ES

1. A process for the manufacture of an antibiotic AAJ-271 complex which comprises culturing <u>Actinomadura parvosata</u> Shearer sp. nov. ATCC 53463 microorganism or an active mutant or derivative thereof in an aqueous nutrient medium containing assimilable sources of nitrogen and carbon under submerged aerobic conditions until a substantial amount of the complex is produced and isolating the complex so produced.

2. The process of claim 1 wherein the culturing of the microorganism occurs at a temperature of from about 15°C to about 42°C and continues for from about 3 to about 8 days.

3. The process of claim 1 which further comprises chromatographically separating one or more of the components of the AAJ-271 complex.

4. The process of claim 1 which further comprises chromatographically separating one or more AAJ-271 antibiotic compounds of the formula (I) :

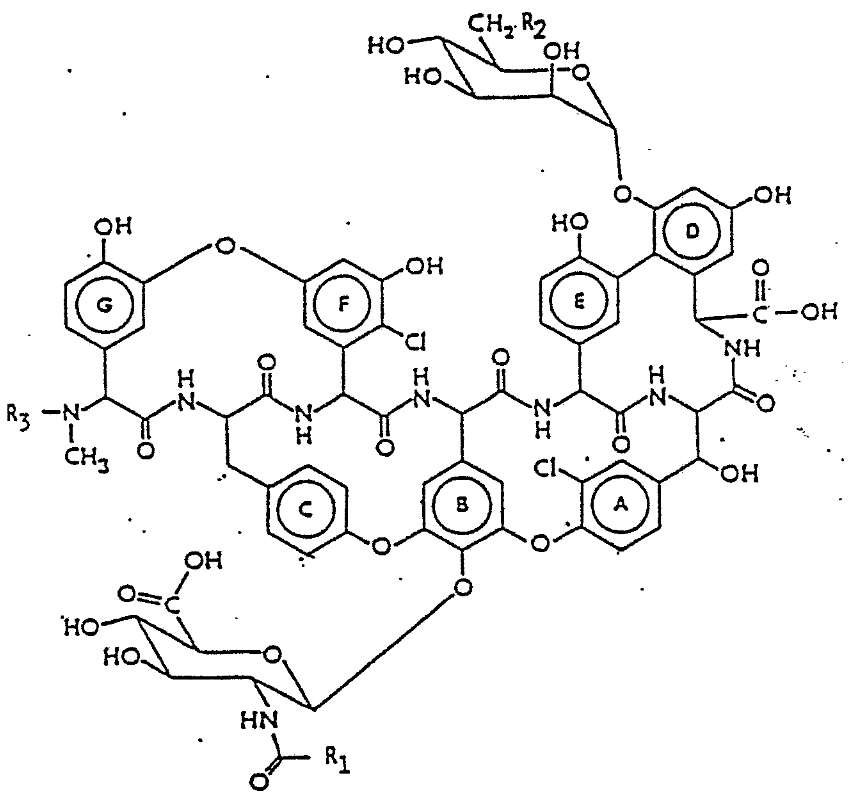

(I)

wherein $R_1$ is $C_{1-15}$alkyl, $R_2$ is OH, and $R_3$ is H or $CH_3$; or a mixture of said compounds, and biologically functional derivatives thereof.

5. The process of Claim 4 for preparing an AAJ-271 antibiotic which is :

AAJ-271$C_1$ wherein $R_1$ is 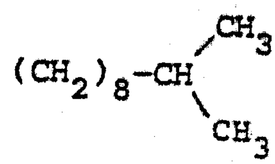, $R_2$ is OH, and $R_3$ is H;

AAJ-271$C_2$ wherein $R_1$ is $(CH_2)_{10}CH_3$, $R_2$ is OH, and $R_3$ is H;

AAJ-271$C_3$ wherein $R_1$ is 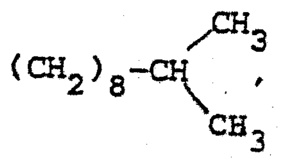, $R_2$ is 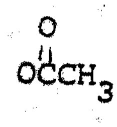, and $R_3$ is H;

AAJ-271$C_4$ wherein $R_1$ is $(CH_2)_{10}CH_3$, $R_2$ is 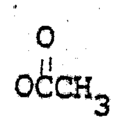, and $R_3$ is H;

AAJ-271X wherein $R_1$ is a straight or branched $C_{11}$ alkyl chain, $R_2$ is OH, and $R_3$ is $CH_3$;

AAJ-271A having a molecular weight of 1702 and containing a hexose, and a N-acyl glucuronic acid moiety with a n-$C_{10}$ acyl group;

AAJ-271$B_1$ having a molecular weight of 1716 and containing a hexose and a N-acyl glucuronic acid moiety with a $C_{11}$ acyl group having terminal iso-branching; or

AAJ-271$B_2$ having a molecular weight of 1716 and containing a hexose and a N-acyl glucuronic acid moiety with a n-$C_{11}$acyl group.

6. A process for the preparation of antibiotic compounds represented by structural formula (II)

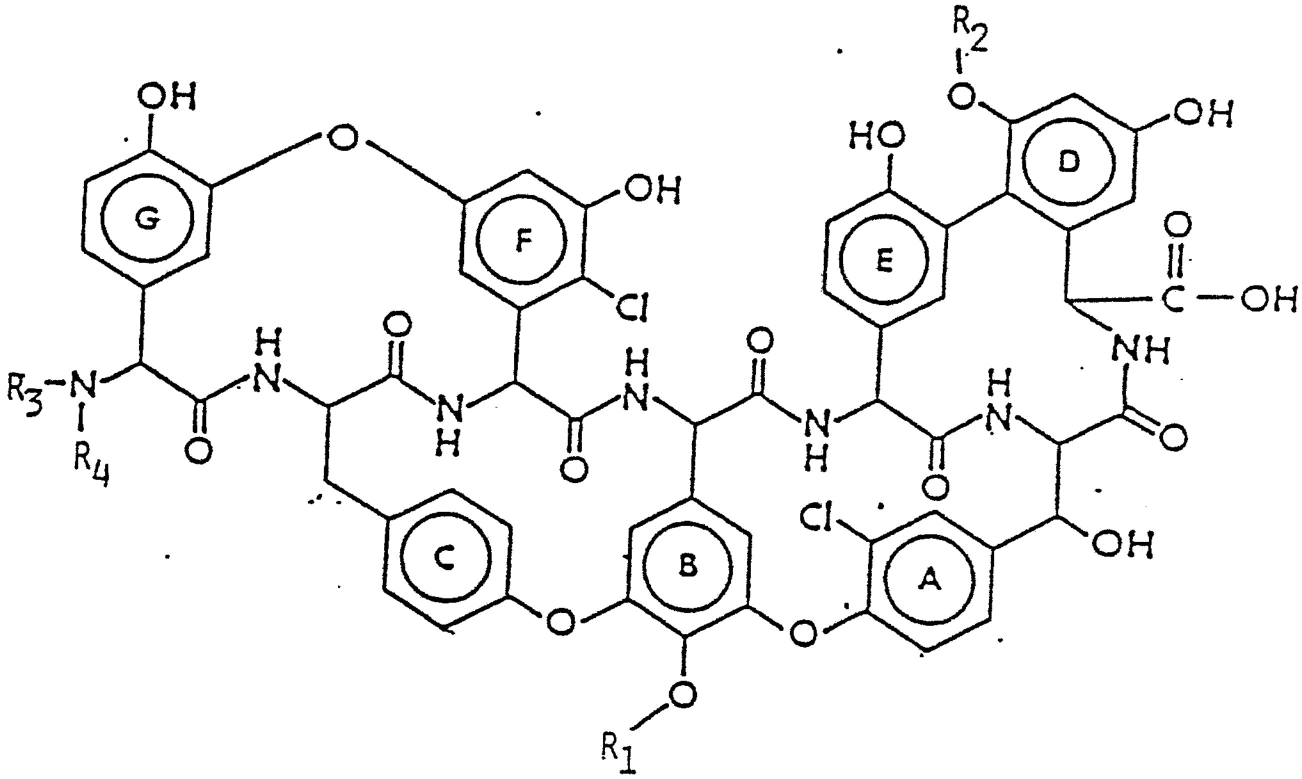

(II)

wherein $R_1$ is hydrogen or a glycolipid radical, $R_2$ is hydrogen or monosaccharide radical, $R_3$ is hydrogen or methyl, and $R_4$ is hydrogen or methyl with the proviso that at least one of $R_1$ or $R_2$ is hydrogen, comprising the partial acid hydrolysis of one or more antibiotics

selected from the group consisting of AAJ-271 complex, and AAJ-271 factors A, $B_1$, $B_2$, $C_1$, $C_2$, $C_3$, $C_4$, or X, until a substantial amount of the compounds are formed and isolating said compounds.

7. The process of claim 6 wherein the hydrolysis is conducted in an aqueous organic solvent with dilute mineral acid at reflux.

8. The process of claim 6 wherein the hydrolysis is conducted in a polar organic solvent with dilute mineral acid at elevated temperature.

9. The process of claim 6 wherein the resulting compounds are isolated by chromatographic means.

10. The process of claim 6 for preparing a compound which is :

AAJ-271 aglycone 1 wherein $R_1$, $R_2$ and $R_3$ are hydrogen and $R_4$ is methyl;

AAJ-271 aglycone 2 wherein $R_1$ and $R_2$ are hydrogen and $R_3$ and $R_4$ are each methyl;

AAJ-271 pseudo-aglycone wherein $R_1$ is hydrogen, $R_2$ is mannosyl, $R_3$ is hydrogen and $R_4$ is methyl;

AAJ-271X pseudo-aglycone 1 wherein $R_1$ is hydrogen, $R_2$ is mannosyl, and $R_3$ and $R_4$ are each methyl;

AAJ-271$C_1$/$C_3$ pseudo-aglycone wherein $R_1$ is N-acyl glucuronic acid with a $C_{12}$ acyl group having terminal iso-branching, $R_2$ and $R_3$ are hydrogen, and $R_4$ is methyl;

AAJ-271$C_2$/$C_4$ pseudo-aglycone wherein $R_1$ is N-acyl glucuronic acid with a n-$C_{12}$ acyl group, $R_2$ and $R_3$ are hydrogen, and $R_4$ is methyl;

AAJ-271A pseudo-aglycone wherein $R_1$ is N-acyl glucuronic acid with a $C_{10}$ acyl group, $R_2$ and $R_3$ are H, and $R_4$ is $CH_3$;

AAJ-271$B_1$ pseudo-aglycone wherein $R_1$ is N-acyl glucuronic acid with a $C_{11}$ acyl group having terminal iso-branching, $R_2$ and $R_3$ are H, and $R_4$ is $CH_3$;

AAJ-271$B_2$ pseudo-aglycone wherein $R_1$ is N-acyl glucuronic acid with a n-$C_{11}$acyl group, $R_2$ and $R_3$ are H, and $R_4$ is $CH_3$;

AAJ-271X pseudo-aglycone 2 wherein $R_1$ is N-acyl glucuronic acid with a $C_{12}$ acyl group, $R_2$ is H, and $R_3$ and $R_4$ are $CH_3$; or

minor pseudo-aglycones wherein $R_1$ is N-acyl glucuronic acid with a $C_{10-12}$ acyl group, $R_2$ is H, and $R_3$ and $R_4$ are $CH_3$.

11. A process for preparing a composition comprising the admixing of a pharmaceutically acceptable carrier and a product as defined in any one of Claims 1, 4 to 6 and 10, or any mixture thereof.

12. A process for preparing an animal feed composition comprising the admixing of an animal feed with a product as defined in any one of Claims 1, 3 to 6 and 10, or any mixture thereof.

13. A process for preparing an animal feed premix composition comprising the admixing of a premix vehicle with a product as defined in any one of Claims 1, 3 to 6 and 10, or any mixture thereof.

14. A method for preventing or treating gram-positive bacterial infections which comprises administering an effective amount of a compound or composition produced by any of the processes of Claims 1, 4 to 6, and 10 to 11 to an infected or susceptible human or animal.

15. A method of improving the growth rate or feed efficiency of meat or milk producing animals which comprises administering a product or composition as produced in any one of Claims 1, 3 to 6, and 10 to 13.

16. A method of increasing propionate production in the rumen or cecum of meat or milk producing animals which comprises administering a product or composition as defined in any one of Claims 1, 3 to 6, and 10 to 13.